(19) Europäisches Patentamt
European Patent Office
Office européen des brevets

(11) EP 2 229 134 B1

(12) **EUROPEAN PATENT SPECIFICATION**

(45) Date of publication and mention of the grant of the patent:
**15.11.2017 Bulletin 2017/46**

(21) Application number: **09701852.7**

(22) Date of filing: **16.01.2009**

(51) Int Cl.:
***A61K 8/44*** (2006.01) ***A61K 8/86*** (2006.01)
***A61K 8/891*** (2006.01) ***A61Q 5/02*** (2006.01)
***A61Q 5/12*** (2006.01) ***A61Q 19/10*** (2006.01)
***A61K 8/46*** (2006.01) ***A61Q 5/00*** (2006.01)
***A61K 8/04*** (2006.01)

(86) International application number:
**PCT/IB2009/050162**

(87) International publication number:
**WO 2009/090617 (23.07.2009 Gazette 2009/30)**

(54) **CONCENTRATED PERSONAL CLEANSING COMPOSITIONS**
KONZENTRIERTE KÖRPERREINIGUNGZUSAMMENSETZUNGEN
COMPOSITION DE SOIN PERSONNEL CONCENTRÉE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **18.01.2008 US 11631 P**
**16.10.2008 US 105989 P**

(43) Date of publication of application:
**22.09.2010 Bulletin 2010/38**

(73) Proprietor: **The Procter & Gamble Company**
**Cincinnati, OH 45202 (US)**

(72) Inventors:
- **KITKO, David, Johnathan**
**Cincinnati**
**OH 45241 (US)**
- **HAMERSKY, Mark, William**
**Indian Springs**
**OH 45011 (US)**
- **HUTTON, Howard, David, III**
**Oregonia**
**OH 45054 (US)**
- **BROWN, David, Clayton**
**Cincinnati**
**OH 45223 (US)**
- **HUTCHINS, Thomas, Allen**
**Cincinnati**
**OH 45245 (US)**
- **JOHNSON, Eric, Scott**
**Hamilton**
**OH 45011 (US)**

(74) Representative: **Briatore, Andrea et al**
**Procter & Gamble Service GmbH**
**IP Department**
**Frankfurter Strasse 145**
**61476 Kronberg im Taunus (DE)**

(56) References cited:
**EP-A1- 1 696 023** **EP-A2- 1 468 666**
**WO-A1-2006/084190** **DE-A1- 4 224 714**
**JP-A- 2002 038 200** **US-A- 3 616 859**
**US-A- 4 024 078** **US-A- 4 753 754**
**US-A- 5 942 485** **US-A1- 2002 151 738**
**US-A1- 2007 119 864** **US-E- R E34 584**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 229 134 B1

**Description**

FIELD OF THE INVENTION

The present invention relates to concentrated personal cleansing compositions having desirable lathering and dissolution properties.

BACKGROUND OF THE INVENTION

Conventional liquid personal cleansing compositions, such as shampoos and body washes, are formulated to contain large amounts of water. The added weight and volume of this water significantly increases the costs of packaging, shipping, storing, and transporting these compositions. In addition, it increases the amount of energy used and the amount of waste generated.

It therefore would be desirable to provide a concentrated liquid personal cleansing composition that contains significantly less water than conventional compositions. Furthermore, it would be desirable for such a composition to have consumer-desired lathering and dissolution attributes.

A liquid personal cleansing composition in a concentrated form, among other things, should providing equal or better performance than traditional liquid personal cleansing compositions at ½ to 1/3 of the usage level of the traditional personal cleansing compositions. Therefore, in a compact form, the amount of carbon-generating resources required to formulate, package, and transport the composition is greatly diminished, thereby lowering the composition’s "carbon footprint."

Formulating a concentrated liquid personal cleansing composition having desirable rheological attributes is not as straight-forward as merely increasing (e.g., doubling or tripling) the level of surfactant present in the formula. The researchers of the present invention have found that merely increasing the level of surfactant used in traditional personal cleansing compositions, such as shampoos, results in a product with less than desirable lathering and dissolution attributes. For instance, the researchers have found that merely fortifying concentrated compositions with more surfactant leads to compositions that have poor lather, poor dissolution, rinse poorly, and are too thick or don’t spread properly on the hands and hair.

Others have attempted to solve these aforementioned problems inherent in making a concentrated personal cleansing composition. Some approaches have involved adding a hydrotrope and/or an organic solvent to the formulation. However, while the addition of these materials may facilitate the formulation of a more highly concentrated composition, they also lead to products with poorer lather and/or dissolution. Concentrated detergent compositions have been described for example in US5942485, EP1696023A1, US4753754 and JP2002-038200A.
Accordingly, the researchers of the present invention endeavored to develop a concentrated liquid personal cleansing composition that does not sacrifice the desired end-use characteristics (e.g., good lather, easy to dissolve, appropriate stiffness and spreading properties) as a trade-off for a more compact form.

Surprisingly, the researchers discovered that concentrated liquid personal cleansing compositions having higher active levels of surfactant, yet still exhibiting good rheology, lathering and dissolution properties, can be made when the surfactant is in an isotropic phase in the composition. Such compositions can comprise greater than about 23% isotropic surfactant and still provide good rheology, lather and dissolution.

SUMMARY OF THE INVENTION

A concentrated personal cleaning composition according to the enclosed claims meets the aforementioned needs.

BRIEF DESCRIPTION OF THE DRAWINGS

Figure 1 shows rheology profiles, as discussed herein, for commercially available shampoo compositions
Figure 2 shows rheology profiles, as discussed herein, for compacted compositions
Figure 3 shows a spreadability profile for a compacted composition

DETAILED DESCRIPTION OF THE INVENTION

The present invention is a concentrated liquid personal cleansing composition suitable for cleansing mammalian keratinous tissue (i.e., human hair, skin, and/or nails).

The compositions herein are substantially free of organic solvent and/or hydrotrope. As used herein, "substantially free" means that the concentration of organic solvent and/or hydrotrope is no more than trace quantities that would be commonly found as an impurity in commercial ingredients. Because different organic solvents and/or hydrotropes can negatively impact rheology at differing magnitudes, the level at which any such material(s) is deemed to be at a "substantially free" concentration will depend upon the particular organic solvent and/or hydrotrope at issue. In one embodiment, the composition comprises less than about 0.5 wt% by weight of the composition of an organic solvent or a hydrotrope individually or combined (if both present), in a particular embodiment from 0 wt% to about 0.5 wt% by weight of the composition of an organic solvent or a hydrotrope, and in some embodiments from 0 wt% to about 0.3 wt% by weight of the composition of an organic solvent or a hydrotrope.

As used herein, the terms "organic solvent" and "hydrotrope" encompass those materials recognized in the art as organic solvents or hydrotropes. Examples of organic solvents include those used in cleansing applications, and can be selected from the group consisting of alcohols, glycols, ethers, ether alcohols, and mixtures thereof. Typical hydrotropes can include cumene, xylene and toluene sulfonates, and mixtures thereof. Both solvent and hydrotrope examples are generally described in McCutcheon's, Detergents and Emulsifiers, North American edition (1986), published by Allured Publishing Corporation; and in McCutcheon's Functional Materials, North American Edition (1992). Surfactant - Isotropic Enabler

The composition of the present invention comprises greater than about 23 wt% by weight of the composition of a surfactant wherein said surfactant is in isotropic form in the composition. In particular embodiments, the composition comprises from about 23 wt% to about 40 wt% by weight of the composition of a surfactant, and in other embodiments from about 30 wt% to about 40 wt% by weight of the composition of a surfactant. In particular embodiments, the surfactant comprises an effective amount of an Isotropic Enabler.

As used herein, the term "Isotropic Enabler" and "Enabler" are used interchangeably and refer to surfactants in an isotropic phase. In one embodiment, an Isotropic Enabler is combined with any other suitable surfactant (e.g., those commonly known in the art).

As used here, the "isotropic phase" of surfactants refers to the state where the individual molecules are associated, but remain in the micellar state. The micelles can have a spherical shape or may be elongated at higher concentrations, but maintain mobility and dynamic exchange and dissolve readily. This is in contrast to the gel phase where the elongated micelles begin to pack in arrays, e.g. long cylindrical structures and have lost their mobility and dynamic exchange capability and therefore do not dissolve or disperse readily upon dilution.

The Isotropic Enablers are carefully chosen based on three key parameters: 1) hydrophobic chainlength, 2) degree of branching in the hydrophobic chainlength, and 3) size of the surfactant head group as dictated by degree of ethoxylation; and combinations of these three parameters. Without being limited by theory, the researchers believe manipulation of these parameters allow the Isotropic Enablers to minimize surfactant phase structure (e.g., control appropriate phase behavior to produce less stringly micelles with no rods or more complex structures). An Isotropic Enabler is a surfactant that can be combined with another surfactant, resulting in a concentrated surfactant mixture that is isotropic in the final composition. The compositions of the present invention comprise an effective amount of Isotropic Enabler. As used herein, an "effective amount" of Isotropic Enabler is at least the minimum amount necessary to combine with another surfactant in order to produce a surfactant mixture that is in isotropic phase in the final composition.

Examples of Isotropic Enabler includes, but are not limited to: branched and non-branched versions of decyl and undecyl alkyl sulfates which are either ethoxylated or non-ethoxylated; decyl alcohol modified lauryl sulfate; paraffin sulfonates with chain lengths ranging from $C_{13}$ to $C_{17}$ sold by the Clariant Company; mixtures of linear and branched-chain alcohol sulfates with carbon chain lengths $C_{12}$ to $C_{17}$ commonly known as LIAL® and NEODOL® alkyl or alcohol sulfates which are ethoxylated or non-ethoxylated; sodium salts of hydroxyethyl-2-dodecyl ether sulfates, or of hydroxyethyl-2-decyl ether sulfates (from Nippon Shokubai Inc., and either or both referred to herein as "NSKK ethoxy sulfate"); monoethoxylated lauryl alkyl sulfates; and mixtures thereof.

Undecyl Sulfates

A suitable Isotropic Enabler includes undecyl sulfates. The undecyl sulfates of the may comprise straight-chain undecyl sulfates having the formula (I) $R_1$-$O(CH_2CHR_3O)_y$-$SO_3M$, branched-chain undecyl sulfates having the general formula (II) $CH_3$-$(CH_2)_z$-$CHR_2$-$CH_2$-$O(CH_2\ CHR_3O)_y$-$SO_3M$, or mixtures thereof, where $R_1$ of formula (I) represents $CH_3(CH_2)_{10}$, $R_2$ of formula (II) represents H or a hydrocarbon radical comprising 1 to 4 carbon atoms such that the sum of the carbon atoms in z of formula (II) and $R_2$ of formula (II) is 8, $R_3$ of formula (II) is H or $CH_3$, y of formulae (I) and (II) is 0 to 7, the average value of y of formulae (I) and (II) is about 1 when y of formulae (I) and (II) is not = 0, and M of formulae (I) and (II) is a mono-valent or di-valent, positively-charged cation. Examples of mono-valent positively charged cations include ammonium, sodium, potassium, triethanolamine cation, and examples of di-valent positively charged cations include magnesium.

"Average value" is understood to mean that whereas the composition may comprise molecules having a value

of y of formulae (I) and (II) other than 1, the average value of y of formulae (I) and (II) of all molecules in the composition is about 1. The undecyl sulfates may comprise from about 70 wt% to about 90 wt% by weight of the undecyl sulfate, and alternatively about 80 wt% by weight of the undecyl sulfate of straight-chain undecyl sulfates and about 10 wt% to about 30 wt% by weight of the undecyl sulfate, and alternatively about 20 wt%, by weight of the undecyl sulfate of branched-chain undecyl sulfates, by weight of the total amount of undecyl sulfates.

The undecyl sulfates of the invention can be prepared by the hydroformylation of 1-decene or internal decenes, for example as described in U.S. 6,706,931 to produce linear and branched primary alcohols which are sulfated with $SO_3$ in a falling film reactor and neutralized to make the alkylsulfuric acid salt, e.g. with sodium hydroxide to produce sodium undecyl sulfate. One example of a suitable alcohol is commercially available as NEODOL® 1 (Shell Oil Co.).

Additionally, the undecyl alcohol can be derived from castor oil via its hydrolysis to obtain ricinoleic acid. Ricinoleic can be pyrolyzed to obtain undecylenic acid. Undecylenic acid can be converted to undecyl alcohol via a series of hydrogenations to obtain undecyl alcohol.

Undecyl Alkoxy Sulfates

A suitable Isotropic Enabler includes undecyl alkoxyl sulfates. The undecyl alkoxy sulfates (undeceth sulfates) may comprise straight-chain and/or branched-chain undecyl alkoxy sulfates having the general formula (II) $CH_3-(CH_2)_z-CHR_2-CH_2-O-(CH_2CHR_3O)_y-SO_3M$, where $R_2$ of formula (II) represents H or a hydrocarbon radical comprising 1 to 4 carbon atoms such that the sum of the carbon atoms in z of formula (II) and $R_2$ is 8, $R_3$ of formula (II) is H or $CH_3$, y of formula (II) is 1 to 7, y of formula (II) has an average value of 1 or less, and M of formula (II) is a monovalent, positively-charged cation such as ammonium, sodium, potassium and/or triethanolamine cation,etc. or divalent salts of magnesium with two surfactant anions.

The undecyl ethoxylates can be prepared by the addition of one molar equivalent of ethylene oxide or less to the undecyl alcohol in the presence of an alkaline catalyst. The resulting material may comprise from about 30 wt% to about 60 wt% by weight of the resulting material of unethoxylated alcohol, and the remaining mixture will consist of a variety of homologues with an ethoxy (EO) content ranging from 1 to 7. This mixture can be sulfated in a falling film reactor with $SO_3$ and neutralized with base, e.g., NaOH, to produce the sodium undecyl alkoxy sulfates. Additionally, mixtures of the undecyl alcohol and undecyl alkoxylate can be blended together and sulfated as above to produce a mixture of undecyl-based surfactants.

Rheological Property Profile

A key parameter of many desired compositions of the present invention is rheology. For perspective, simply concentrating surfactant compositions by increasing the surfactant level typically leads to poor rheology exemplified by highly stiff flow and stringiness. This stiff flow and stringiness lead to a product which is very difficult to spread on the hands and hair/skin. We have defined a rheology method to mimic the behavior of spreading the cleaning composition on hands or hair. During this action, there is a transient response where the shampoo is at rest in the hand after dosing and then is instantaneously sheared to a high rate (ca. 1000 $sec^{-1}$). The resulting response of the shampoo is critical in the desirability of the shampoo from a hand feel and spreading perspective.

The first key parameter is the instant shear peak @ 1000 sec-1. This measures the behavior that you experience as you begin to rub the product in your hands or between hands and hair/skin.

The second parameter is the rheology measure 1.2 seconds after this initial shear. In our preferred embodiments, this value will be substantially lower indicating that the product will spread readily upon input of energy via rubbing/spreading.

The third parameter is the rheology at 2 seconds after the instant shear where we want the product to remain thin and easily spread through the hair. We believe the combination of these three parameters provide guidance in delivering acceptable product delivery and use for the consumers.

From looking at the current data on the compacted shampoos and commercial benchmarks, the peak shape seems to be a good discriminating tool. Figure 1 shows two commercial products, not according to the present invention, for exemplification of the testing parameters above for the rheology profile. The upper curve shows a peak initially (indicated in Figure 1 by A) and the value remains relatively high throughout the test indicated in Figure 1 by B. However, the lower curve exhibits virtually no peak (indicated in Figure 1 by C) and the level remains low throughout the test as indicated in Figure 1 by D. The product from the upper curve has very poor consumer acceptance for spreading and hand feel. The lower curve has acceptable hand feel.

Figure 2 shows the rheology profile of compositions which are compacted compositions, such as shampoos, exhibit a higher initial peak, but the peak rapidly decays down to low level, which avoids consumer negatives on hand feel. It is clear that consumer acceptability has much to do with the total rheological profile rather than a specific point. The Rheology profile method attempts to capture the defined key parameters as shown in Figure 2 in Zone 1 (first key

parameter), Zone 2 (second key parameter) or Zone 3 (third key parameter). The peak is depicted in the plot in Figure 2 in Zone 1 (first key parameter) shown as A in Figure 2 and represents the initial resistance to motion by the liquid. The rapid decay (or lack thereof) the peak is observed in Zone 2 (second key parameter) shown as B in Figure 2. The continued resistance is contained in Zone 3 (third key parameter) shown as C in Figure 2. The curves labeled as 1 and 2 are acceptable rheology profiles for compacted compositions, while the curve labeled as 3 is not an acceptable rheology profile for compacted compositions.

We have attempted to characterize this profile with three data points and with consumer studies, we have found that a prototype has acceptable hand spreading when the first key parameter is a peak in this test is greater than the second or third key parameter, but then rapidly decays (second key parameter) less than the first key parameter, and continues to remain (third key parameter) low about the same or slightly less then the second key parameter.

The present invention targets materials that have a high initial peak (to meet the definition of a concentrated shampoo) but then quickly breakdown under shear of hand rubbing - therefore values which are low after the peak and after continued rubbing are desired.

In some embodiments, the instant shear peak is from about 0.2 to about 8 Pa-s (Pascal-seconds), and in particular embodiments from about 0.8 to about 8 Pa-s, and in still other embodiments, greater than about 0.8 Pa-sec.

In particular embodiments, the value at 1.2 sec (seconds) after the start of the instant shear measurement procedure is less than about 0.8 Pa-s, in some embodiments less than about 0.6 Pa-s, and in others less than about 0.4 Pa-s. In still other embodiments, the value at 1.2 sec is from about 0 to about 0.8 Pa-s, and in alternate embodiments from about 0.05 to about 0.8 Pa-s.

In some embodiments, the value at 2 sec after the start of the instant shear measurement procedure is less than about 0.8 Pa-s, in others less than about 0.4 Pa-s, and in still others less than about 0.2 Pa-s. In still other embodiments, the value at 2 sec after the start of the instant shear is from about 0 to about 0.8 Pa-s, and in others from about 0.05 to about 0.8 Pa-s.

Spreadability With Respect To Rheology Profile

Another element to consumer desirability of a composition is how the composition, such as a shampoo, spreads through wet hair and on wet hands. This relates to how rapidly the shampoo's structure changes with a small addition of water. The spreadability can be correlated to viscosity and the shear rate at which the structure breaks.

Depending on the surfactant and polymer choices, as well as salt levels and other contributing factors, the spreading parameter (c1 * c2), as discussed in the Method below, is seen to vary from levels from around 180 Pa to as high as 1000 Pa. Testing, according to this methodology discussed herein, has shown those formulations according to the present invention with a spreading parameter around 200 Pa or less are acceptable for spreading with wet hands and hair.

In this case, the spreading parameter would be the product of 16.3 Pa-s and 41.2 $s^{-1}$ (= 671 Pa). This prototype happens to be a poorly rated for spreading by consumers. For acceptable spreading behavior our analysis and consumer tests have shown that the product of c1 and c2 parameter needs to be in the vicinity of 200 Pa or less.

Bubble Size

Consumers have come to identify consumer products cleaning functionality with bubble size in the foam/lather generated in use of the product. In personal cleansing products smaller bubble sizes are typically associated with products providing benefits beyond cleaning, e.g. hair or skin conditioning. It is therefore desirable to tailor the in use bubble size derived during use of the cleaning product. The researchers have discovered that judicious use of longer chain length surfactant, such as ceteareth sulfate or Neodol® 67 sulfate, as minor constituents in the surfactant system can give rise to bubble size manipulation during use of the product to signal these benefits. This can produce small bubbles where the lather has a creamy feel, in contrast to large bubbles that do not produce a creamy-feeling lather.

In some embodiments, the composition has a lather bubble size of less than about 0.025 $mm^2$, and in others from about 0.005 $mm^2$ to about 0.025 $mm^2$, and in still other embodiments from about 0.005 $mm^2$ to about 0.02 $mm^2$.

Additional Surfactants

Any suitable additional surfactant can be combined with an Isotropic Enabler to form an isotropic surfactant mixture and utilized in the composition of the present invention. Non-limiting examples of suitable surfactants are disclosed in U.S. 5,624,666, in McCutcheon's, Detergents and Emulsifiers, North American edition (1986), published by Allured Publishing Corporation; and in McCutcheon's Functional Materials, North American Edition (1992). Desirable additional surfactants include anionic surfactants and optionally other co-surfactants.

Anionic Surfactants

Additional anionic surfactants suitable for use herein include alkyl and alkyl ether sulfates of the formula (III) $ROSO_3M$ and formula (IV) $RO(C_2H_4O)_xSO_3M$, wherein R of formulae (III) and (IV) is alkyl or alkenyl of from about 8 to about 18 carbon atoms, x of formula (IV) is 1 to 10, and M of formulae (III) and (IV) is a water-soluble cation such as ammonium, sodium, potassium, and triethanolamine cation or salts of the divalent magnesium ion with two anionic surfactant anions. The alkyl ether sulfates may be made as condensation products of ethylene oxide and monohydric alcohols having from about 8 to about 18 carbon atoms. The alcohols can be derived from fats, e.g., coconut oil, palm oil, palm kernel oil, or tallow, or can be synthetic.
Examples of additional anionic surfactants suitable for use herein include, but are not limited to, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium trideceth sulfate, sodium tridecyl sulfate, sodium methyl lauroyl taurate, sodium methyl cocoyl taurate, sodium lauroyl isethionate, sodium cocoyl isethionate, sodium laurethsulfosuccinate, sodium laurylsulfosuccinate, sodium tridecyl benzene sulfonate, sodium dodecyl benzene sulfonate, and mixtures thereof.

Co-surfactants.

Co-surfactants are materials which are combined with the Isotropic Enabler and optionally anionic surfactants to enhance lather volume and/or to modify lather texture. Typically these materials can be selected from a variety of families of structures including, but not limited to, amphoteric, zwitterionic, cationic, and nonionic. They are typically used with anionic surfactants in a weight ratio of 1:20 to 1:4, more preferably in the 1:12 to 1:7 weight ratio.
Amphoteric surfactants suitable for use herein include, but are not limited to derivatives of aliphatic secondary and tertiary amines in which the aliphatic radical can be straight or branched chain and wherein one substituent of the aliphatic substituents contains from about 8 to about 18 carbon atoms and one contains an anionic water solubilizing group, e.g., carboxy, sulfonate, sulfate, phosphate, or phosphonate. Examples include sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. 2,658,072, N-higher alkyl aspartic acids such as those produced according to the teaching of U.S. 2,438,091, and the products described in U.S. 2,528,378, and mixtures thereof. The family of amphoacetates derived from the reaction of sodium chloroacetate with amidoamines to produce alkanoyl amphoacetates are particularly effective, e.g. lauryolamphoacetate, and the like.
Zwitterionic surfactants suitable for use herein include, but are not limited to derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one substituent contains an anionic group, e.g., carboxy, sulfonate, sulfate, phosphate, or phosphonate. Other zwitterionic surfactants suitable for use herein include betaines, including high alkyl betaines such as coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyethyl betaine, and mixtures thereof. The sulfobetaines may include coco dimethyl sulfopropyl betaine, stearyl dimethyl sulfopropyl betaine, lauryl dimethyl sulfoethyl betaine, lauryl bis-(2-hydroxyethyl) sulfopropyl betaine and mixtures thereof. Also suitable amphoteric surfactants include amidobetaines and amidosulfobetaines, wherein the $RCONH(CH_2)_3$ radical, wherein R is a $C_{11}$-$C_{17}$ alkyl, is attached to the nitrogen atom of the betaine are also useful in this invention.
Nonionic co-surfactants typically used in the present composition for enhancing lather volume or texture include water soluble materials like lauryl dimethylamine oxide, cocodimethylamine oxide, cocoamidopropylamine oxide, laurylamidopropyl amine oxide, etc. or alkylpolyethoxylates like laureth-4 to laureth-7 and water insoluble components such as cocomonoethanol amide, cocodiethanol amide, lauroylmonoethanol amide, alkanoyl isopropanol amides, and fatty alcohols like cetyl alcohol and oleyl achohol, and 2-hydroxyalkyl methyl ethers and the like.
Suitable materials include 1,2-alkylepoxides, 1,2-alkanediols, branched or straight chain alkyl glyceryl ethers (e.g., as disclosed in EP 1696023A1), 1,2-alkylcyclic carbonates, and 1,2-alkyl cyclicsulfites, particularly those wherein the alkyl group contains 6 to 14 carbon atoms in linear or branched configuration. Other examples include the alkyl ether alcohols derived from reacting $C_{10}$ or $C_{12}$ alpha olefins with ethylene glycol (e.g., hydroxyethyl-2-decyl ether, hydroxyethyl-2-dodecyl ether), as can be made according to the teachings of U.S. 5,741,948; U.S. 5,994,595; U.S. 6,346,509;

and U.S. 6,417,408.

Other preferred nonionic surfactants may be selected from the group consisting of glucose amides, alkyl polyglucosides, sucrose cocoate, sucrose laurate, alkanolamides, ethoxylated alcohols and mixtures thereof.

In one embodiment the nonionic surfactant is selected from the group consisting of glyceryl monohydroxystearate, isosteareth-2, trideceth-3, hydroxystearic acid, propylene glycol stearate, PEG-2 stearate, sorbitan monostearate, glyceryl laurate, laureth-2, cocamide monoethanolamine, lauramide monoethanolamine, and mixtures thereof.

In a particular embodiment, the co-surfactant is selected from the group consisting of Cocomonoethanol Amide, Cocoamidopropyl Betaine, Laurylamidopropyl Betaine, Cocobetaine, lauryl betaine, lauryl amine oxide, sodium lauryl amphoacetate; alkyl glyceryl ethers, alkyl-di-glyceryl ethers, 1,2-alkyl cyclic sulfites, 1,2-alkyl cyclic carbonates, 1,2-alkyl-epoxides, alkyl glycidylethers, and alkyl-1,3-dioxolanes, wherein the alkyl group contains 6 to 14 carbon atoms in linear or branched configuration; 1,2- alkane diols where the total carbon content is from 6 to 14 carbon atoms linear or branched, methyl-2-hydroxy-decyl ethers, hydroxyethyl-2-dodecyl ether, hydroxyethyl-2-decyl ether, and mixtures thereof.

Cationic surfactants may be derived from amines that are protonated at the pH of the formulation, e.g. bis-hydroxyethyl lauryl amine, lauryl dimethylamine, lauroyl dimethyl amidoproplyl amine, cocoylamidopropyl amine, and the like. The cationic surfactants may also be derived from fatty quaternary ammonium salts such as lauryl trimethylammonium chloride and lauroylamidopropyl trimethyl ammonium chloride.

The composition of the present invention may comprise from about 0.5 wt% to about 10 wt%, alternatively from about 0.5 wt% to about 5 wt%, and alternatively from about 1 wt% to about 3 wt% by weight of the composition of at least one suitable co-surfactant.

Conditioning Agent

The composition of the present invention can comprise a conditioning agent, and in some embodiments at least about 0.05 wt% by weight of the composition of a conditioning agent. In particular embodiments, the composition comprises from about 0.05 wt% to about 10 wt%, and in other embodiments from about 0.05 wt% to about 2 wt%, in alternate embodiments from about 0.5% to about 10 wt% by weight of the composition of a conditioning agent, and in still other embodiments from about 0.5 wt% to about 6 wt% by weight of the composition of a conditioning agent. Conditioning agents can include, for example, cationic polymer, large and small particle silicone (e.g., small particle silicone of less than 0.1 microns), and oils. Any suitable conditioning agent can be used. Conditioning agents include any material which is used to give a particular conditioning benefit to hair and/or skin.

Cationic Polymer

The conditioning agents for use in the present composition may contain a cationic polymer. A suitable cationic polymer will have a cationic charge density of at least about 0.3 meq/gm, typically at least about 0.5 meq/gm, commonly at least about 0.7 meq/gm, but also generally less than about 7 meq/gm, typically less than about 6.5 meq/gm, at the pH of intended use of the composition. The pH of intended use of the composition generally ranges from about pH 3 to about pH 9, typically from about pH 4 to about pH 8. A suitable cationic polymer will generally have an average molecular weight ranging from about 1,000 to about 10,000,000, typically from about 10,000 to about 5,000,000, commonly about 20,000 to about 2,000,000.

The term "charge density", as used herein, refers to the ratio of the number of positive charges on a polymer to the molecular weight of said polymer.

Suitable cationic polymers for use in the compositions of the present invention can contain cationic nitrogen-containing moieties such as quaternary ammonium or cationic protonated amino moieties. The cationic protonated amines can be primary, secondary, or tertiary amines (typically secondary or tertiary), depending upon the particular species and the selected pH of the composition. Any anionic counterions can be used in association with the cationic polymers so long as the polymers remain soluble in water, in the composition, or in a coacervate phase of the composition, and so long as the counterions are physically and chemically compatible with the components of the composition or do not otherwise unduly impair product performance, stability or aesthetics. Non-limiting examples of such counterions include halides (e.g., chloride, fluoride, bromide, iodide), sulfate and methylsulfate.

Non-limiting examples of such polymers are described in the CTFA Cosmetic Ingredient Dictionary, 3rd edition, edited by Estrin, Crosley, and Haynes, (The Cosmetic, Toiletry, and Fragrance Association, Inc., Washington, D.C. (1982)). Non-limiting examples of suitable cationic polymers include copolymers of vinyl monomers having cationic protonated amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone or vinyl pyrrolidone.

Suitable cationic protonated amino and quaternary ammonium monomers, for inclusion in the cationic polymers

of the composition herein, include vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts.

Other suitable cationic polymers for use in the compositions include copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16); copolymers of 1-vinyl-2-pyrrolidone and dimethylaminoethyl methacrylate (referred to in the industry by CTFA as Polyquaternium-11); cationic diallyl quaternary ammonium-containing polymers, including, for example, dimethyldiallylammonium chloride homopolymer, copolymers of acrylamide and dimethyldiallylammonium chloride (referred to in the industry by CTFA as Polyquaternium 6 and Polyquaternium 7, respectively); amphoteric copolymers of acrylic acid including copolymers of acrylic acid and dimethyldiallylammonium chloride (referred to in the industry by CTFA as Polyquaternium 22), terpolymers of acrylic acid with dimethyldiallylammonium chloride and acrylamide (referred to in the industry by CTFA as Polyquaternium 39), and terpolymers of acrylic acid with methacrylamidopropyl trimethylammonium chloride and methylacrylate (referred to in the industry by CTFA as Polyquaternium 47). Suitable cationic substituted monomers are the cationic substituted dialkylaminoalkyl acrylamides, dialkylaminoalkyl methacrylamides, and combinations thereof. These suitable monomers conform to the formula (V):

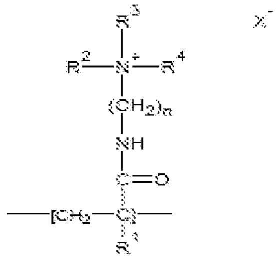

Formula (V)

wherein $R^1$ of formula (V) is hydrogen, methyl or ethyl; each of $R^2$, $R^3$, and $R^4$ of formula (V) are independently hydrogen or a short chain alkyl having from about 1 to about 8 carbon atoms, typically from about 1 to about 5 carbon atoms, commonly from about 1 to about 2 carbon atoms; n is an integer having a value of from about 1 to about 8, typically from about 1 to about 4; and X of formula (V) is a counterion. The nitrogen attached to $R^2$, $R^3$, and $R^4$ of formula (V) may be a protonated amine (primary, secondary, or tertiary), but is typically a quaternary ammonium wherein each of $R^2$, $R^3$, and $R^4$ of formula (V) are alkyl groups, a non-limiting example of which is polymethyacrylamidopropyl trimonium chloride, available under the trade name Polycare 133, from Rhone-Poulenc, Cranberry, N.J., U.S.A.

Other suitable cationic polymers for use in the composition include polysaccharide polymers, such as cationic cellulose derivatives and cationic starch derivatives. Suitable cationic polysaccharide polymers include those which conform to the formula (VI):

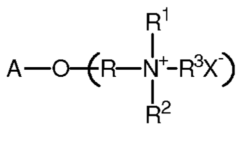

Formula (VI)

wherein A of formula (VI) is an anhydroglucose residual group, such as a starch or cellulose anhydroglucose residual; R of formula (VI) is an alkylene oxyalkylene, polyoxyalkylene, or hydroxyalkylene group, or combination thereof; $R^1$, $R^2$, and $R^3$ of formula (X) independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in $R^1$, $R^2$, and $R^3$ of formula (VI)) typically being about 20 or less; and X of formula (VI) is an anionic counterion as described hereinbefore.

Suitable cationic cellulose polymers are salts of hydroxyethyl cellulose reacted with trimethyl ammonium substituted epoxide, referred to in the industry (CTFA) as Polyquaternium 10 and available from Amerchol Corp. (Edison, N.J., USA) in their Polymer LR, JR, and KG series of polymers. Other suitable types of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp., under the tradename Polymer LM-200.

Other suitable cationic polymers include cationic guar gum derivatives, such as guar hydroxypropyltrimonium chloride, specific examples of which include the Jaguar series commercially avaialable from Rhone-Poulenc Incorporated and the N-Hance series commercially available from Aqualon Division of Hercules, Inc. Other suitable cationic polymers include quaternary nitrogen-containing cellulose ethers, some examples of which are described in U.S. 3,962,418. Other suitable cationic polymers include copolymers of etherified cellulose, guar and starch, some examples of which are described in U.S. 3,958,581. When used, the cationic polymers herein are either soluble in the composition or are soluble in a complex coacervate phase in the composition formed by the cationic polymer and the detersive surfactant components described hereinbefore. Complex coacervates of the cationic polymer can also be formed with other charged materials in the composition.

Copolymers - copolymer may be comprises of two cationic monomer or a nonionic and cationic monomers.

Nonionic Monomer Unit

The synthetic copolymers comprise the nonionic monomer unit represented by the following formula (VII):

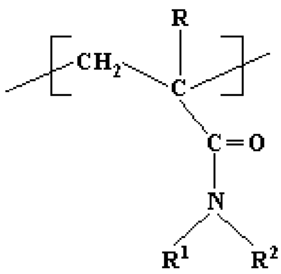

formula (VII)

where R of formula (VII) is H or $C_{1-4}$ alkyl; and $R^1$ and $R^2$ of formula (VII) are independently selected from the group consisting of H, $C_{1-4}$ alkyl, $CH_2OCH_3$, $CH_2OCH_2CH(CH_3)_2$, and phenyl, or together are $C_{3-6}$ cycloalkyl.

In one embodiment, nonionic monomer unit is acrylamide (AM), i.e., where R, $R^1$, and $R^2$ of formula (VII) are all H as shown below in formula (VIII):

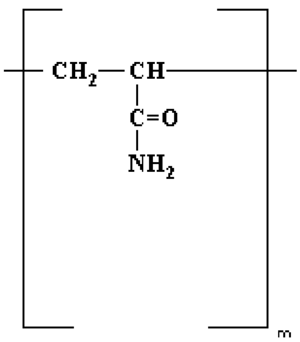

Formula (VIII)

m of formula (VIII) is equal to 1.

Another preferred nonionic monomer unit is methacrylamide (MethAM), i.e., where R of formula (VII) is $C_1$ alkyl, and $R^1$ and $R^2$ of formula (VII) are each H respectively:

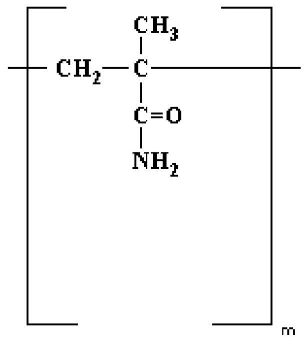

Formula (IX)

m of formula (IX) is equal to 1.

However, the other acrylamide derivatives within the scope of the formula (IX) set out above are also contemplated to be part of the present invention where polyacrylamide and copolymers using acrylamide monomers are useful.

The nonionic monomer portion of the synthetic copolymers is present in an amount from about 50 wt% to about 99.5 wt% by weight of the total copolymer. Preferably, this amount is from about 70 wt% to about 99 wt %, still more preferably from about 80 wt% to about 99 wt% by weight of the copolymer.

Cationic Monomer Unit

The copolymers also comprise the cationic monomer unit represented by Formula (X):

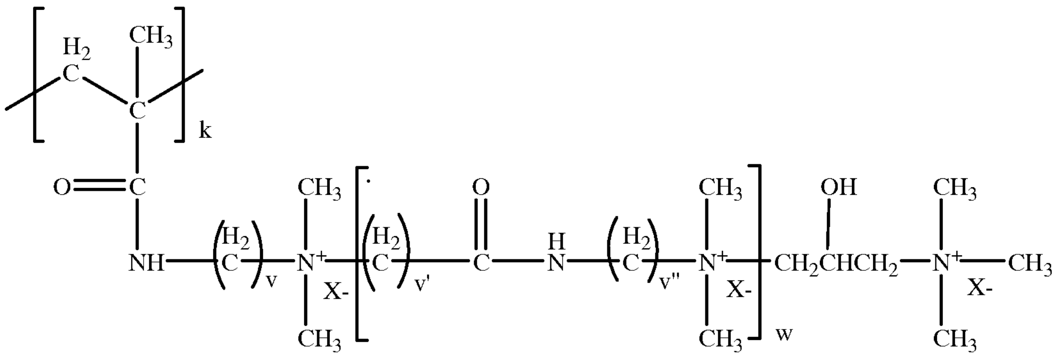

Formula (X)

where k of formula (X) is equal to 1, each of v, v', and v" of formula (X) is independently an integer of from 1 to 6, w of formula (X) is zero or an integer of from 1 to 10, and X- of formula (X) is an anion.

In one embodiment, a structure of formula (X) is present where k = 1, v = 3 and w = 0, z = 1 and $X^-$ is $Cl^-$, to form the following formula (XI):

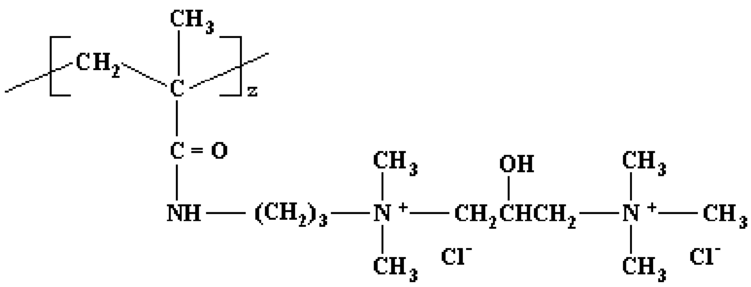

Formula (XI)

The above structure may be referred to as diquat.

Yet another embodiment is achieved by the structure of formula (X) formed wherein v and v" are each 3, v' = 1, w =1, y = 1 and $X^-$ is $Cl^-$ such as:

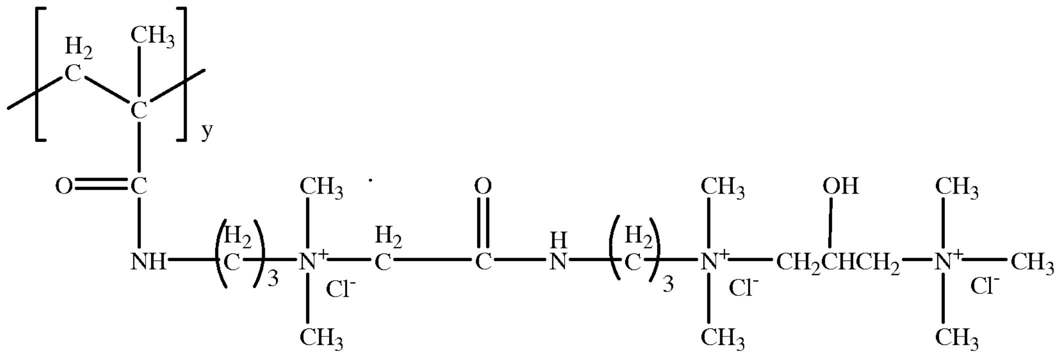

Formula (XII)

The above structure may be referred to as triquat.

Suitable cationic monomers can be made by, for example, the methods described in U.S. Patent Application Publication No. 2004/0010106 A1.

Silicones

A conditioning agent useful in the compositions of the present invention is typically an insoluble, non-volatile silicone conditioning agent in particle form. The silicone conditioning agent particles may comprise volatile silicone, non-volatile silicone, or combinations thereof. The silicone conditioning agent particles may comprise a silicone fluid conditioning agent and may also comprise other ingredients, such as a silicone resin, to improve silicone fluid deposition efficiency or enhance glossiness of the hair.

Non-limiting examples of suitable silicone conditioning agents, and optional suspending agents for the silicone, are described in U.S. Reissue Pat. No. 34,584, U.S. 5,104,646, and U.S. 5,106,609. The silicone conditioning agents for use in the compositions of the present invention generally have a viscosity, as measured at 25°C, from about 20 to about 2,000,000 centistokes ("csk"), typically from about 1,000 to about 1,800,000 csk, commonly from about 50,000 to about 1,500,000 csk, typically from about 100,000 to about 1,500,000 csk.

The dispersed silicone conditioning agent particles typically have a number average particle diameter ranging from about 0.005μm to about 50μm. For small particle application to hair, the number average particle diameters typically range from about 0.01μm to about 4μm, commonly from about 0.01μm to about 2μm, generally from about 0.01μm to about 0.5μm. For larger particle application to hair, the number average particle diameters typically range from about 4μm to about 50μm, commonly from about 6μm to about 30μm, generally from about 9μm to about 20μm, typically from about 12μm to about 18μm.

Silicone oils

Silicone fluids include silicone oils, which are flowable silicone materials having a viscosity, as measured at 25°C, less than 1,000,000 csk, typically from about 5 csk to about 1,000,000 csk, commonly from about 100 csk to about 600,000 csk. Suitable silicone oils for use in the compositions of the present invention include polyalkyl siloxanes, polyaryl siloxanes, polyalkylaryl siloxanes, polyether siloxane copolymers, and mixtures thereof. Other insoluble, non-volatile silicone fluids having hair conditioning properties may also be used.

Amino and Cationic Silicones

Cationic silicone fluids suitable for use in the compositions of the present invention include, but are not limited to, those which conform to the general formula (XIII):

$$(R_1)_aG_{3-a}Si\text{-}(\text{-}OSiG_2)_n\text{-}(\text{-}OSiG_b(R_1)_{2-b})_m\text{-}O\text{-}SiG_{3-a}(R_1)_a$$

wherein G of formula (XIII) is hydrogen, phenyl, hydroxy, or a $C_1$-$C_8$ alkyl, typically a methyl; a of formula (XIII) is 0 or an integer having a value from 1 to 3, typically 0; b of formula (XIII) is 0 or 1, typically 1; n of formula (XIII) is a number from 0 to 1,999, typically from 49 to 499; m of formula (XIII) is an integer from 1 to 2,000, typically from 1 to 10; the sum

of n and m of formula (XIII) is a number from 1 to 2,000, typically from 50 to 500; $R_1$ of formula (XIII) is a monovalent radical conforming to the general formula (XIV) $C_qH_{2q}L$, wherein q of formula (XIV) is an integer having a value from 2 to 8 and L of formula (XIV) is selected from the following groups:

formula (XV): $-N(R_2)CH_2-CH_2-N(R_2)_2$

formula (XVI): $-N(R_2)_2$

formula (XVII): $-N(R_2)_3A^-$

formula (XVIII): $-N(R_2)CH_2-CH_2-NR_2H_2A^-$

wherein $R_2$ of formulae (XV) - (XVIII) is hydrogen, phenyl, benzyl, or a saturated hydrocarbon radical, typically an alkyl radical from about $C_1$ to about $C_{20}$, and A of formulae (XV) - (XVIII) is a halide ion.

A suitable cationic silicone corresponding to formula (XIII) is the polymer known as "trimethylsilylamodimethicone", which is shown below in formula (XIX):

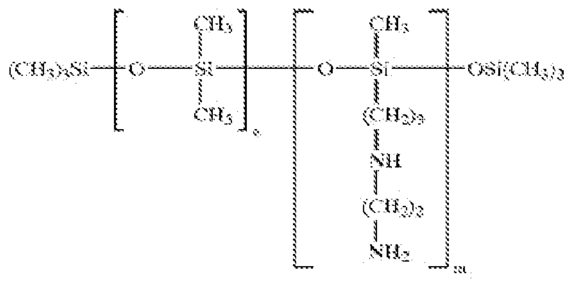

Formula (XIX)

Other silicone cationic polymers which may be used in the compositions of the present invention are represented by the general formula (XX):

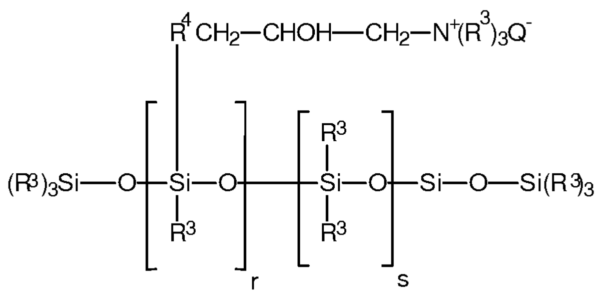

Formula (XX)

wherein $R^3$ of formula (XX) is a monovalent hydrocarbon radical from $C_1$ to $C_{18}$, typically an alkyl or alkenyl radical, such as methyl; $R^4$ of formula (XX) is a hydrocarbon radical, typically a $C_1$ to $C_{18}$ alkylene radical or a $C_{10}$ to $C_{18}$ alkyleneoxy radical, commonly a $C_1$ to $C_8$ alkyleneoxy radical; Q of formula (XX) is a halide ion, typically chloride; r of formula (XX) is an average statistical value from 2 to 20, typically from 2 to 8; s of formula (XX) is an average statistical value from 20 to 200, typically from 20 to 50. A suitable polymer of this class is known as UCARE SILICONE ALE 56™, available from Union Carbide.

Silicone gums

Other silicone fluids suitable for use in the compositions of the present invention are the insoluble silicone gums. These gums are polyorganosiloxane materials having a viscosity, as measured at 25°C, of greater than or equal to

1,000,000 csk. Silicone gums are described in U.S. Pat. No. 4,152,416; Noll and Walter, Chemistry and Technology of Silicones, New York: Academic Press (1968); and in General Electric Silicone Rubber Product Data Sheets SE 30, SE 33, SE 54 and SE 76. Specific non-limiting examples of silicone gums for use in the compositions of the present invention include polydimethylsiloxane, (polydimethylsiloxane) (methylvinylsiloxane) copolymer, poly(dimethylsiloxane) (diphenylsiloxane)(methylvinylsiloxane) copolymer and mixtures thereof.

High Refractive Index Silicones

Other non-volatile, insoluble silicone fluid conditioning agents that are suitable for use in the compositions of the present invention are those known as "high refractive index silicones," having a refractive index of at least about 1.46, typically at least about 1.48, commonly at least about 1.52, typically at least about 1.55. The refractive index of the polysiloxane fluid will generally be less than about 1.70, typically less than about 1.60. In this context, polysiloxane "fluid" includes oils as well as gums.

Silicone resins

Silicone resins may be included in the silicone conditioning agent of the compositions of the present invention. These resins are highly cross-linked polymeric siloxane systems. The cross-linking is introduced through the incorporation of trifunctional and tetrafunctional silanes with monofunctional or difunctional, or both, silanes during manufacture of the silicone resin.

Organic conditioning oils

The conditioning component of the compositions of the present invention may also comprise from about 0.05 wt% to about 3 wt%, typically from about 0.08 wt% to about 1.5 wt%, commonly from about 0.1 wt% to about 1 wt%, of at least one organic conditioning oil, either alone or in combination with other conditioning agents, such as the silicones.

Hydrocarbon oils

Suitable organic conditioning oils for use as conditioning agents in the compositions of the present invention include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils typically are from about $C_{12}$ to about $C_{19}$. Branched chain hydrocarbon oils, including hydrocarbon polymers, typically will contain more than 19 carbon atoms.

Polyolefins

Organic conditioning oils for use in the compositions of the present invention can also include liquid polyolefins, typically liquid poly-$\alpha$-olefins, commonly hydrogenated liquid poly-$\alpha$-olefins. Polyolefins for use herein are prepared by polymerization of $C_4$ to about $C_{14}$ olefenic monomers, typically from about $C_6$ to about $C_{12}$.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents. Typical hydrogenated $\alpha$-olefin monomers include, but are not limited to, 1-hexene to 1-hexadecenes, 1-octene to 1-tetradecene, and mixtures thereof.

Fatty Esters

Other suitable organic conditioning oils for use as the conditioning agent in the compositions of the present invention include, but are not limited to, fatty esters having at least 10 carbon atoms. These fatty esters include esters with hydrocarbyl chains derived from fatty acids or alcohols (e.g. mono-esters, polyhydric alcohol esters). The hydrocarbyl radicals of the fatty esters hereof may include other compatible functionalities, such as amides and alkoxy moieties (e.g., ethoxy or ether linkages, etc.).

Other Conditioning Agents

Also suitable for use in the compositions herein are the conditioning agents described by the Procter & Gamble

Company in U.S. 5,674,478, and U.S. 5,750,122. Also suitable for use herein are those conditioning agents described in U.S. 4,529,586, U.S. 4,507,280, U.S. 4,663,158, U.S. 4,197,865, U.S. 4,217, 914, U.S. 4,381,919 and U.S. 4,422, 853.

The composition has a lather index greater than about 0.75, and in some embodiments from about 0.75 to about 1.5. The composition has a dissolution value of less than about 200 seconds ("sec"), and in one embodiment from about 10 to about 200 sec, and in an alternate embodiment from about 100 to about 200 sec.

Optional Ingredients

The concentrated personal care composition may include a broad range of additional components, depending on the product form and its intended use and end benefit. Non-limiting examples of optional components for use in compositions of the present invention are described in U.S. 6,335,312, issued to Coffindaffer et al. and include conventional personal care polymers (deposition polymers, styling polymers, dispersed phase polymers), anti dandruff agents, and combinations thereof.

Furthermore, additional components can include anti-static agents, humectants and emollients, suspending agents, viscosity modifiers which increase viscosity, pigments, dyes, pearlescent aids, foam boosters, antimicrobial agents, pediculocides, pH adjusting agents, perfumes, anti-oxidants, preservatives, chelating agents, sequestrants, proteins, skin care actives, sunscreens, UV absorbers, vitamins and other aesthetic components such as essential oils, panthenol and derivatives (e.g. ethyl panthenol), pantothenic acid and its derivatives, xanthenes (such as caffeine), clove oil, menthol, camphor, eucalyptus oil, eugenol, menthyl lactate, witch hazel distillate, allantoin, bisabalol, dipotassium glycyrrhizinate, derivatives of any of the foregoing and combinations thereof.

Any other suitable optional component can also be included in the personal care composition of the present invention, such as those ingredients that are conventionally used in given product types. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the concentrated personal care composition.

Foam Boosters

Suitable foam boosters may be included to increase the foaming ability of the composition, especially when used with a foaming pump. One embodiment is the use of sodium 2-(2-hydroxyalkyloxy)acetate sold under the name Beaulight SHAA by the company Sanyo.

Anti-Dandruff Actives

The compositions of the present invention may also contain an anti-dandruff agent. Suitable, non-limiting examples of anti-dandruff particulates include: pyridinethione salts, zinc carbonate, azoles, such as ketoconazole, econazole, and elubiol, selenium sulfide, particulate sulfur, salicylic acid and mixtures thereof. A typical anti-dandruff particulate is pyridinethione salt. Such anti-dandruff particulate should be physically and chemically compatible with the components of the composition, and should not otherwise unduly impair product stability, aesthetics or performance.

Pyridinethione Salts

Pyridinethione anti-dandruff particulates, especially 1-hydroxy-2-pyridinethione salts, are suitable particulate anti-dandruff agents for use in compositions of the present invention. The concentration of pyridinethione anti-dandruff particulate typically ranges from about 0.01 wt% to about 5 wt%, by weight of the composition, generally from about 0.1 wt% to about 3 wt%, commonly from about 0.1 wt% to about 2 wt%. Suitable pyridinethione salts include those formed from heavy metals such as zinc, tin, cadmium, magnesium, aluminum and zirconium, generally zinc, typically the zinc salt of 1-hydroxy-2-pyridinethione (known as "zinc pyridinethione" or "ZPT"), commonly 1-hydroxy-2-pyridinethione salts in platelet particle form, wherein the particles have an average size of up to about 20μ, typically up to about 5μ, commonly up to about 2.5μ. Salts formed from other cations, such as sodium, may also be suitable. Pyridinethione anti-dandruff agents are described, for example, in U.S. 2,809,971; U.S. 3,236,733; U.S. 3,753,196; U.S. 3,761,418; U.S. 4,345,080; U.S.. 4,323,683; U.S. 4,379,753; and. U.S. 4,470,982.

Anti-microbial Actives

In addition to the anti-dandruff active selected from polyvalent metal salts of pyrithione, the present invention may further comprise one or more anti-fungal or anti-microbial actives in addition to the metal pyrithione salt actives. Suitable anti-microbial actives include coal tar, sulfur, whitfield's ointment, castellani's paint, aluminum chloride, gentian

violet, octopirox (piroctone olamine), ciclopirox olamine, undecylenic acid and it's metal salts, potassium permanganate, selenium sulphide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. Typical anti-microbials include itraconazole, ketoconazole, selenium sulphide and coal tar.

Azoles

Azole anti-microbials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole anti-microbial active is included in an amount from about 0.01% to about 5%, typically from about 0.1% to about 3%, and commonly from about 0.3% to about 2%, by weight of the composition. Especially common for use herein is ketoconazole.

Selenium Sulfide

Selenium sulfide is a particulate anti-dandruff agent suitable for use in the anti-microbial compositions of the present invention, effective concentrations of which range from about 0.1 wt% to about 4 wt%, by weight of the composition, typically from about 0.3 wt% to about 2.5 wt%, commonly from about 0.5 wt% to about 1.5 wt%. Selenium sulfide is generally regarded as a compound having one mole of selenium and two moles of sulfur, although it may also be a cyclic structure that conforms to the general formula $Se_xS_y$, wherein x + y = 8. Average particle diameters for the selenium sulfide are typically less than 15μm, as measured by forward laser light scattering device (e.g. Malvern 3600 instrument), typically less than 10μm. Selenium sulfide compounds are described, for example, in U.S. 2,694,668; U.S. 3,152,046; U.S. 4,089,945; and U.S. 4,885,107.

Sulfur

Sulfur may also be used as a particulate anti-microbial/anti-dandruff agent in the anti-microbial compositions of the present invention. Effective concentrations of the particulate sulfur are typically from about 1 wt% to about 4 wt%, by weight of the composition, typically from about 2 wt% to about 4 wt%.

Keratolytic Agents

The present invention may further comprise one or more keratolytic agents such as Salicylic Acid.

• Zinc-Containing Layered Material

In an embodiment of the present invention, the composition may include an effective amount of a zinc-containing layered material. Preferred embodiments of the present invention include from about 0.001% to about 10% of a zinc-containing layered material; more preferably from about 0.01% to about 7%; more preferably still from about 0.1% to about 5%.

Examples of zinc-containing layered materials useful in certain embodiments of the present invention include the following:

Zinc-containing layered structures are those with crystal growth primarily occurring in two dimensions. It is conventional to describe layer structures as not only those in which all the atoms are incorporated in well-defined layers, but also those in which there are ions or molecules between the layers, called gallery ions (A.F. Wells "Structural Inorganic Chemistry" Clarendon Press, 1975). Zinc-containing layered materials (ZLM's) may have zinc incorporated in the layers and/or be components of the gallery ions.

Many ZLM's occur naturally as minerals. Common examples include hydrozincite (zinc carbonate hydroxide), basic zinc carbonate, aurichalcite (zinc copper carbonate hydroxide), rosasite (copper zinc carbonate hydroxide) and many related minerals that are zinc-containing. Natural ZLM's can also occur wherein anionic layer species such as clay-type minerals (e.g., phyllosilicates) contain ion-exchanged zinc gallery ions. All of these natural materials can also be obtained synthetically or formed in situ in a composition or during a production process.

Another common class of ZLM's, which are often, but not always, synthetic, is layered doubly hydroxides, which

are generally represented by the formula $[M^{2+}{}_{1-x}M^{3+}(OH)_2]^{x+}\ A^{m-}{}_{x/m}\cdot nH_2O$ and some or all of the divalent ions ($M^{2+}$) would be represented as zinc ions (Crepaldi, EL, Pava, PC, Tronto, J, Valim, JB J. Colloid Interfac. Sci. 2002, 248, 429-42).

Yet another class of ZLM's can be prepared called hydroxy double salts (Morioka, H., Tagaya, H., Karasu, M, Kadokawa, J, Chiba, K Inorg. Chem. 1999, 38, 4211-6). Hydroxy double salts can be represented by the general formula $[M^{2+}{}_{1-x}M^{2+}{}_{1+x}(OH)_{3(1-y)}]^{+}\ A^{n-}{}_{(1=3y)/n}\cdot nH_2O$ where the two metal ion may be different; if they are the same and represented by zinc, the formula simplifies to $[Zn_{1+x}(OH)_2]^{2x+}$ 2x $A^{-}\cdot nH_2O$. This latter formula represents (where x=0.4) common materials such as zinc hydroxychloride and zinc hydroxynitrate. These are related to hydrozincite as well wherein a divalent anion replace the monovalent anion. These materials can also be formed in situ in a composition or in or during a production process.

These classes of ZLM's represent relatively common examples of the general category and are not intended to be limiting as to the broader scope of materials which fit this definition.

Commercially available sources of basic zinc carbonate include Zinc Carbonate Basic (Cater Chemicals: Bensenville, IL, USA), Zinc Carbonate (Shepherd Chemicals: Norwood, OH, USA), Zinc Carbonate (CPS Union Corp.: New York, NY, USA), Zinc Carbonate (Elementis Pigments: Durham, UK), and Zinc Carbonate AC (Bruggemann Chemical: Newtown Square, PA, USA).

Basic zinc carbonate, which also may be referred to commercially as "Zinc Carbonate" or "Zinc Carbonate Basic" or "Zinc Hydroxy Carbonate", is a synthetic version consisting of materials similar to naturally occurring hydrozincite. The idealized stoichiometry is represented by $Zn_5(OH)_6(CO_3)_2$ but the actual stoichiometric ratios can vary slightly and other impurities may be incorporated in the crystal lattice

It has been found, in accordance with an embodiment of the present invention, that anti-dandruff efficacy can be dramatically increased in topical compositions by the combination of an effective amount of a zinc-containing layered material wherein the zinc-containing layered material has a specified zinc lability within a surfactant system. Zinc lability is a measure of the chemical availability of zinc ion. Soluble zinc salts that do not complex with other species in solution have a relative zinc lability, by definition, of 100%. The use of partially soluble forms of zinc salts and/or incorporation in a matrix with potential complexants generally lowers the zinc lability substantially below the defined 100% maximum.

Labile zinc is maintained by choice of an effective zinc-containing layered material or formation of an effective zinc-containing layered material in-situ by known methods.

It has been found, in accordance with an embodiment of the present invention, that anti-dandruff efficacy can be dramatically increased in topical compositions by the use of polyvalent metal salts of pyrithione, such as zinc pyrithione, in combination with zinc-containing layered materials. Therefore an embodiment of the present invention provides topical compositions with improved benefits to the skin and scalp (e.g., improved antidandruff efficacy).

An embodiment of the present invention provides a stable composition for zinc-containing layered material dispersion where the zinc source resides in a particulate form. It has been shown to be challenging to formulate aqueous systems containing a zinc-containing layered material, due to the zinc-containing layered material's unique physical and chemical properties. Zinc-containing layered material may have a high density (approximately 3 g/cm$^3$), and needs to be evenly dispersed throughout the product and so it will not aggregate or settle. Zinc-containing layered material also has a very-reactive surface chemistry as well as the propensity to dissolve in systems with pH values below 6.5.

A zinc-containing layered material with a solubility of less than 25% will have a measurable % soluble zinc value below a threshold value determined by the weight percent and molecular weight of the zinc compound. The theoretical threshold value can be calculated by the following equation:

$$\frac{0.25 * wt.\%\ \text{Zn Compound in Composition} * \text{moles of Zinc in Compound} * 65.39\ (\text{MW of Zn})}{\text{MW of Zn Compound}}$$

In an embodiment of the present invention, wherein the composition comprises a ZLM, the pH may be greater than about 6.5; further, the pH may be in a range from about 6.5 to about 12, preferably from about 6.8 to about 9.5, more preferably from about 6.8 to about 8.5.

Method for Assessment of Zinc Lability in Zinc-Containing Products

Zinc lability is a measure of the chemical availability of zinc ion. Soluble zinc salts that do not complex with other species in solution have a relative zinc lability, by definition, of 100%. The use of partially soluble forms of zinc salts and/or incorporation in a matrix with potential complexants generally lowers the zinc lability substantially below the defined 100% maximum.

Zinc lability is assessed by combining a diluted zinc-containing solution or dispersion with the metallochromic dye xylenol orange (XO) and measurement of the degree of color change under specified conditions. The magnitude of color formation is proportional to the level of labile zinc. The procedure developed has been optimized for aqueous

surfactant formulations but may be adapted to other physical product forms as well.

A spectrophotometer is used to quantify the color change at 572 nm, the wavelength of optimum color change for XO. The spectrophotometer is set to zero absorbance at 572nm utilizing a product control as close in composition to the test product except excluding the potentially labile form of zinc. The control and test products are then treated identically as follows. A 50μl product sample is dispensed into ajar and 95 ml of deaerated, distilled water are added and stirred. 5mL of a 23mg/mL xylenol orange stock solution at pH 5.0 is pipetted into the sample jar; this is considered time 0. The pH is then adjusted to 5. 50±0.01 using dilute HCl or NaOH. After 10.0 minutes, a portion of the sample is filtered (0.45μ) and the absorbance measured at 572nm. The measured absorbance is then compared to a separately measured control to determine the relative zinc lability (zero TO 100%). The 100% lability control is prepared in a matrix similar to the test products but utilizing a soluble zinc material (such as zinc sulfate) incorporated at an equivalent level on a zinc basis. The absorbance of the 100% lability control is measured as above for the test materials. The relative zinc lability is preferably greater than about 15%, more preferably greater than about 20%, and even more preferably greater than about 25%.

Using this methodology, the below examples demonstrate a material (basic zinc carbonate) that has intrinsically high lability in an anionic surfactant system compared to one (ZnO) with low intrinsic lability.

| | Relative Zinc Lability (%) | Relative Zinc Lability (%) | Lability Benefit |
|---|---|---|---|
| | In Water | In Simple Surfactant System[1] | |
| Zinc Oxide | 86.3 | 1.5 | NO |
| Basic zinc carbonate | 100 | 37 | YES |
| Simple surfactant system: 6% sodium lauryl sulfate | | | |

Particle Size of ZLM

In an embodiment of the present invention, it is has been found that a smaller particle size is inversely proportional to relative zinc lability

D(90) is the particle size which corresponds to 90% of the amount of particles are below this size. In an embodiment of the present invention, the zinc-containing layered material may have a particle size distribution wherein 90% of the particles are less than about 50 microns. In a further embodiment of the present invention, the zinc-containing layered material may have a particle size distribution wherein 90% of the particles are less than about 30 microns. In yet a further embodiment of the present invention, the zinc-containing layered material may have a particle size distribution wherein 90% of the particles are less than about 20 microns.

Particle Size Determination Method

Particle size analyses on zinc oxide and hydrozincite raw materials are done using the Horiba LA-910 Particle Size Analyzer. The Horiba LA-910 instrument uses the principles of low-angle Fraunhofer Diffraction and Light Scattering to measure the particle size and distribution in a dilute solution of particles. Samples of these two types of raw materials are predispersed in a dilute solution of Lauryl Polyether Alcohol and mixed before introduction to the instrument. On introduction the sample is further diluted and allowed to circulate in the instrument before a measurement is taken. After measurement a calculation algorithm is used to process the data that results in both a particle size and distribution. D(50) is the median particle size or the particle size which corresponds to 50% of the amount of particles are below this size. D(90) is the particle size which corresponds to 90% of the amount of particles are below this size.
D(10) is the particle size which corresponds to 10% of the amount of particles are below this size.

Using this methodology, the below examples demonstrate the relationship between particle size and relative zinc lability for basic zinc carbonate.

| Source | As received/milled[1] | Particle Size (μ)[2] | Relative Zinc Lability (%) |
|---|---|---|---|
| Elementis | As received | 4.5 | 51.6 |
| Elementis | Milled | 1.0 | 67.1 |
| Brüggemann | As received | 4.5 | 56.9 |

(continued)

| Source | As received/milled[1] | Particle Size (μ)[2] | Relative Zinc Lability (%) |
|---|---|---|---|
| Brüggemann | Milled | 1.0 | 76.4 |
| [1] Milling method<br>[2] Particle size Determination | | | |

Surface Area of ZLM

In an embodiment of the present invention, there may be a direct relationship between surface area and relative zinc lability.

Increased particle surface area generally increases zinc lability due to kinetic factors. Particulate surface area can be increased by decreasing particle size and/or altering the particle morphology to result in a porous particle or one whose overall shape deviates geometrically from sphericity.

In an embodiment of the present invention, the basic zinc carbonate may have a surface area of greater than about 10 $m^2$/gm. In a further embodiment, the basic zinc carbonate may have a surface area of greater than about 20 $m^2$/gm. In yet a further embodiment of the present invention, the basic zinc carbonate may have a surface area of greater than about 30 $m^2$/gm.

Surface Area Methodology

Surface area analysis is done using the Micromeritics Auto Pore IV. The Micromeritics Auto Pore IV uses the principles of capillary law governing penetration of a non-wetting liquid, more specifically mercury, into small pores to measure the total pore surface area. This law is expressed by the Washburn equation:

$$D = (1/P)\ 4\gamma \cos \varphi$$

where D is pore diameter, P is the applied pressure, $\gamma$ the surface tension of mercury, and $\varphi$ the contact angle between the mercury and the sample. The Washburn equation assumes that all pores are cylindrical. Representative surface area measurements were conducted on basic zinc carbonate and are described below.

Results

| Sample | Surface Area ($m^2$/g) |
|---|---|
| Brüggemann Zinc Carbonate[1] | 50.57 |
| Elementis Zinc Carbonate[2] | 38.0 |
| 1. Commercially available as Zinc Carbonate AC<br>2. Commerically available as Zinc Carbonate | |

.Coordinating Compound Having a Log Zn Binding Constant

In a further embodiment of the present invention, the composition further comprises a coordinating compound with a Log Zn binding constant in a range sufficient to maintain zinc bioavailability. Preferably, such a coordinating compound has a Log Zn binding constant less than about 6, preferably less than about 5, more preferable less than about 4, and greater than about -0.5. Preferably such a coordinating compound is an organic acid, strong mineral acid, or coordinating species. Preferred examples of such coordinating compounds include the following (respective Log Zn Binding Constant indicated in parenthesis): EDTA (16.5), EDDS (13.5), EDDA (11.1), NTA (10.7), Xylenol Orange (10.3), Cysteine (9.1), Cystine (6.7), Aspartic Acid (Aspartate) (5.9), Glycine (5.0), Citric Acid (Citrate) (4.8), Glutamic Acid (4.5), Methionine (4.4), Arginine (4.2), Carbonic Acid (Carbonate) (3.9), Ornithine (3.8), Tatronic Acid (Tartrate) (3.2), Malic Acid (Malate) (2.9), Malonic Acid (Malonate) (2.9), Tartaric Acid (Tartrate) (2.7), Adipic Acid (Adipate) (2.6),Phosphoric Acid (Phosphate) (2.4), Phthalic Acid (Phthalate) (2.2), Glycolic Acid (Glycolate) (2.0), Lactic Acid (Lactate) (1.9), Succinic Acid (Succinate) (1.8), Acetic Acid (Acetate) (1.0), Sulfuric Acid (Sulfate) (0.9), Boric Acid (Borate) (0.9), Formic Acid (Formate) (0.6), Chloride (-0.3).

Pyrithione or a Polyvalent metal salt of Pyrithione

In an embodiment, the present invention may comprise pyrithione or a polyvalent metal salt of pyrithione. Any form of polyvalent metal pyrithione salts may be used, including platelet and needle structures. Preferred salts for use herein include those formed from the polyvalent metals magnesium, barium, bismuth, strontium, copper, zinc, cadmium, zirconium and mixtures thereof, more preferably zinc. Even more preferred for use herein is the zinc salt of 1-hydroxy-2-pyridinethione (known as "zinc pyrithione" or "ZPT"); more preferably ZPT in platelet particle form, wherein the particles have an average size of up to about 20μm, preferably up to about 5μm, more preferably up to about 2.5μm.

Pyridinethione anti-microbial and anti-dandruff agents are described, for example, in U.S. 2,809,971; U.S. 3,236,733; U.S. 3,753,196; U.S. 3,761,418; U.S. 4,345,080; U.S. 4,323,683; U.S. 4,379,753; and U.S. 4,470,982.

It is further contemplated that when ZPT is used as the anti-microbial particulate in the anti-microbial compositions herein, that an additional benefit of hair growth or re-growth may be stimulated or regulated, or both, or that hair loss may be reduced or inhibited, or that hair may appear thicker or fuller.

Zinc pyrithione may be made by reacting 1-hydroxy-2-pyridinethione (i.e., pyrithione acid) or a soluble salt thereof with a zinc salt (e.g. zinc sulfate) to form a zinc pyrithione precipitate, as illustrated in U.S. 2,809,971.

Preferred embodiments include from about 0.01 wt% to about 5 wt% of a pyrithione or polyvalent metal salt of a pyrithione; more preferably from about 0.1 wt% to about 2 wt%.

In embodiments having a zinc-containing layered material and a pyrithione or polyvalent metal salt of pyrithione, the ratio of zinc-containing layered material to pyrithione or a polyvalent metal salt of pyrithione is preferably from 5:100 to 10:1; more preferably from about 2:10 to 5:1; more preferably still from 1:2 to 3:1.

Additional Anti-microbial Actives

Additional anti-microbial actives of the present invention may include extracts of melaleuca (tea tree) and charcoal. The present invention may also comprise combinations of anti-microbial actives. Such combinations may include octopirox and zinc pyrithione combinations, pine tar and sulfur combinations, salicylic acid and zinc pyrithione combinations, elubiol and zinc pyrithione combinations, elubiol and salicylic acid combinations, octopirox and climbasole combinations, and salicylic acid and octopirox combinations, and mixtures thereof.

Other Optional Components

The compositions of the present invention may contain also vitamins and amino acids such as: water soluble vitamins such as vitamin $B_1$, $B_2$, $B_6$, $B_{12}$, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

The compositions of the present invention may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names.

The compositions of the present invention may also contain antimicrobial agents which are useful as cosmetic biocides and antidandruff agents including: water soluble components such as piroctone olamine, water insoluble components such as 3,4,4'- trichlorocarbanilide (trichlosan), triclocarban and zinc pyrithione.

<u>Methods of Making</u>

Any suitable method of making the concentrated personal care compositions of the present invention may be used. In one embodiment, Isotropic Enabler surfactant is blended with the other components of the concentrated personal care compositions, according to standard methods known in the art.

The typical procedure used for a shampoo would be to combine the Isotropic Enabler paste or mixtures thereof with water, add the desired water soluble co-surfactant and finish the composition by the addition of preservatives, pH control agents, perfume, and salts. If a water insoluble co-surfactant is desired the surfactant and water mixture can be heated to a suitable temperature, typically above the melting point of the water insoluble co-surfactant, to facilitate its incorporation. In some cases, the surfactant paste is combined with the co-surfactant as above and diluted with water to a target level commensurate to achieving the final activity. Rheology modifiers which can increase the viscosity can be added at this point followed by conditioning agents, e.g. silicones or silicone emulsions or other oils, cationic polymers from polymer premixes, perfumes, pearlizing agents or opacifiers, perfumes, and preservatives. Appropriate mixing steps to insure homogeneity are used as needed. The product is finished by the addition of pH control agents, and salts.

In a particular embodiment, the concentrated personal care composition comprises a surfactant wherein said surfactant comprises Isotropic Enabler. The Isotropic Enabler can be prepared as an isotropic, fluid paste in a falling film reactor with typically available neutralization equipment at or above 25% active surfactant, and in some embodiments from about 25% to about 40% active surfactant.

In a particular embodiment where the composition comprises about three-times the level of surfactant that is typically present in Typical Conditioning Shampoo Formulations (i.e., surfactant concentrations for compositions having about one-third the typical shampoo weight or volume level), one or more of the surfactants are derived from higher activity (i.e., concentration) lamellar phase pastes (SLE(1)S, $C_{11}S$, $C_{11}E(1)S$, $C_{13\text{-}15}$paraffin sulfonate). These materials can be made for a number of the Isotropic Enablers, e.g., SLE(1)S, $C_{11}E(1)S$, $C_{13\text{-}15}$ paraffin sulfonate, NSKK ethoxy sulfate, as flowable fluids. Higher activity isotropic pastes of $C_{11}S$ (35%) and SLS containing an enriched level (about 20% $C_{10}$ sulfate) of $C_{10}$ (38%) can be made to facilitate these formulations as well.

In a typical procedure the isotropic paste will be combined with the co-surfactant, preservatives, and the desired quantity of lamellar phase paste. This will be mixed on a speed mixer, such as a Flak Tek, until uniform. Pearlizer dispersion (EGDS), cationic polymer, and perfume are added and mixed until a uniform mixture is achieved. Finally, silicone emulsion, additional preservatives and pH control agents are mixed in with a lower energy input to achieve a gentle agitation for the desired final product mixture.

The present process also provides a method to make decyl alcohol modified lauryl sulfate. The researchers have discovered that the addition of decyl alcohol at levels from about 5 wt% to about 25 wt% can enable the production of higher activity, thin, isotropic surfactant pastes. In a typical process, about 20 wt% by weight decanol is added to lauryl alcohol (a mixture of $C_{12}$, $C_{14}$, and $C_{16}$ linear primary alcohols) and the mixture is fed to a Falling Film Sulfator. There it is combined with $SO_3$ gas and the sulfation reaction proceeds to completion and the resulting acid mix is neutralized with NaOH (50%) in a high shear mixer with sufficient water added to achieve the desired activity (e.g., 38% surfactant concentration).

<u>Methods of Use</u>

The compositions of the present invention may be used for cleansing and conditioning mammalian keratinous tissue such as hair and/or skin, and provide rapid lathering and/or rinseability. The method for cleansing and conditioning the hair may comprise the steps of: a) wetting the hair with water, b) applying an effective amount of the shampoo composition to the hair, and c) rinsing the shampoo composition from the hair using water. These steps can be repeated as many times as desired to achieve the desired cleansing and conditioning benefit.

According to yet another embodiment of the present invention, a method of providing a stable personal care foam is provided, comprising the step of dispensing from a suitable pump dispenser a composition according to the first embodiment. Non-limiting examples of suitable pump dispensers include those described in WO 2004/078903, WO 2004/078901, and WO 2005/078063. A desired foam-generating dispenser comprises a nozzle from which the composition is dispensed whereby the composition enters a foam-generating dispenser via a dip tube. A liquid piston creates a suction which draws the composition into a liquid chamber and thereby primes the foam-generating dispenser. Meanwhile, an air chamber and an air piston are also primed, and when the activator is depressed, both the air from the air chamber and the composition from the liquid chamber are turbulently forced into the mixing chamber and past a first mesh and a second mesh. As the turbulent air/ composition mixture is forced past the first mesh a first, rough foam is generated, which becomes more fine and even after passing through the second mesh and the third mesh. These meshes may have the same, or different pore sizes. Also, additional meshes may also be employed, as desired.

Preferred foam-generating dispensers useful herein include: T8900, OpAd FO, 8203, and 7512 series Roamers from Afa-Polytek, Helmond, The Netherlands; T1, F2, and WR-F3 series roamers from Airspray International, Inc., Alkmaar, The Netherlands or North Pompano Beach, Florida, U.S.A.; TS-800 and Mixor series roamers from Saint-Gobain Calmar, Inc., City of Industry, California, U.S.A.; pump roamers and squeeze roamers from Daiwa Can Company, Tokyo, Japan; TS1 and TS2 series roamers from Guala Dispensing USA, Inc., Hillsborough, New Jersey, U.S.A.; and YT-87L-FP, YT-87L-FX, and YT-97 series roamers from Yoshino Kogyosho Co., Ltd., Tokyo, Japan. Also see the foam-generating dispensers discussed in the Japanese-language publications Food & Package, (2001) vol. 42, no. 10, pp 609-13; Food & Package, (2001) vol. 42, no. 11, pp 676-79; and Food & Package, (2001) vol. 42, no. 12, pp 732-35.

The composition may have a viscosity prior to dispensing of from about 10 to about 100 centipoise (cps), in another embodiment from about 10 to about 60 cps, and in another embodiment from about 10 to about 30 cps.

The amount of the composition applied, the frequency of application and the period of use will vary widely depending upon the purpose of application, the level of components of a given composition and the level of cleansing desired. For example, when the composition is applied to the hair, effective amounts generally range from about 0.25g to 25 g, or alternatively, an amount sufficient to contact most or all of the hair with the composition.

H. Article of Manufacture

The present invention provides for an article of manufacture comprising one or more compositions described herein, and a communication directing a consumer to apply the composition to keratinous tissue to produce a cleansing effect, a rapidly lathering foam, a rapidly rinsing foam, a clean rinsing foam, and combinations thereof. The communication may be printed material attached directly or indirectly to packaging that contains the composition. Alternatively, the communication may be an electronic or a broadcast message that is associated with the article of manufacture. Alternatively, the communication may describe at least one possible use, capability, distinguishing feature and/or limitation of the article of manufacture.

Analytical Methods

Spreadability With Respect To Rheology Profile Method:

10g of shampoo is added to a vial to which 0.5g of deionized water is added and stirrer. The mixture is allowed to equilibrate overnight. This mixture is then subjected to a rheological test using a 50 mm cone and plate fixture and gap of 51 micron, a steady shear rate sweep is conducted. The viscosity is measured as a function of shear rate. The data is then fitted using the Ellis model, a well-known 3 parameter viscosity-shear rate model. The outcome of the fit provides the zero-shear viscosity (c1), a critical shear rate (c2), and a power law related parameter (c3). The product of first two parameters (c1 and c2) is then used as an indicator of wet hand/hair spreadability. A representative plot is shown in Figure 1 with the fitting included. Data from the experiment are shown as filled circles and the Ellis fit is drawn as a line.

Switch Lather Test Method

The switch lather test method is designed to evaluate the lather ease and volume for shampoo products. Switches of Oriental virgin hair, flat construction, 15g/25.4 cm (10 inches), are treated uniformly with 0.1g of artificial sebum from hexane solution to provide a realistic soil level. In the lather evaluation the switch is first wet with tap water (37.8°C (100°F), 7-10gpg hardness) and deliquored to a water content of 1g $H_2O$/1g hair. 0.75 ml of product is applied to the center of the switch; the lower portion of the switch is then rubbed over the product on the hair with 5 strokes in circular motion to distribute the product evenly. This is followed by 40 strokes with a back and forth motion.

Lather ease is determined by the number of strokes required for lather to bloom. After the 40, back and forth, strokes are completed the lather is collected from the operators gloves and placed into a graduated cylinder. The lather remaining in the hair is gathered in one downward stroke (squeeging) and is added to the initial amount. The total lather volume is recorded.

Three runs are made on each test product and the mean of the three values is calculated and used to represent the lather volume per treatment. High (Typical Clarifying Shampoo Formulation) and mid-range (Typical Conditioning Shampoo Formulations) market products are typically included as benchmark controls. A prototype shampoo's lather volume is indexed to the high lather control as 1.0.

Lather Bubble Size Method

Hair switches made from medium brown virgin hair are pre-soiled with artificial sebum from a hexane solution to leave 0.5% sebum by weight on hair. These switches are wet with warm water to a level of 1g $H_2O$/1g of hair. For Typical Conditioning Shampoo Formulations, apply at 0.5% on weight of hair, and the switch is lathered. For the more concentrated shampoos of the present invention the level is adjusted to reflect the compaction factor, e.g., for a 2X compaction the level is reduced to 0.25%.

After lathering the foam is collected and quickly placed (within 10 seconds) on a glass prism surface so that it fully wets the surface. The bubble size is determined by an image analysis system that employs an optics system comprising a Sony B&W progressive scan camera and Computar lens capable of producing a high quality image of a 4mm by 3mm field of view. The bubbles on only the prism surface become visible and are analyzed by a Global Lab Image system, V/2 v 3.7, and their average size is recorded. The test is performed in triplicate and the average value is reported along with the standard deviation.

Wet and Dry Conditioning Test Method

This test method is designed to allow for a subjective evaluation of the basic performance of Typical Conditioning Shampoo Formulations for both wet combing and dry combing efficacy. The control treatments are a Typical Clarifying

Shampoo Formulation that employs only surfactants and has no conditioning materials present and this same shampoo used in the washing process followed by the application of a Typical Conditioning Shampoo Formulations. These treatments allow for easy differentiation of performance of a set prototype conditioning shampoos. In a typical test 3 to 5 separate formulations can be assessed for their performance. The substrate is virgin brown hair obtainable from a variety of sources that is screened to insure uniformity and lack of meaningful surface damage.

Treatment Procedure

Five 4 gram, 20.3 cm (8 inch) length switches are combined in a hair switch holder, wet for ten seconds with manipulation with water at 40°C and typical hardness (9-10 gpg) to insure complete and even wetting. The switch is deliquored lightly and product is applied uniformly over the length of the combined switches from one inch below the holder towards the end at a level of 0.1 g Typical Conditioning Shampoo Formulations per one gram of dry hair (0.1 g/g of hair or 2 g for 20 g hair). For more concentrated compositions of the present invention the usage level is reduced to 0.05 g/g of hair. The switch combo is lathered by a rubbing motion typical of that used by consumers for 30 seconds and rinsed with water flowing at 5.68 L/min. (1.5 gal/min.) at 40°C (with the hair being manipulated) to insure completeness for 30 seconds. This step is repeated once. Where conditioner is applied, it is applied in the same way on the switch combo as shampoo above, manipulated throughout the switch combo and rinsed thoroughly with manipulation. The switches are deliquored lightly, separated from each other, hung on a rack so that they are not in contact and detangled with a wide tooth comb.

Grading Procedures

For wet combing evaluations using trained graders, the switches are separated on the rack into the five sets with one switch from each treatment included in the grading set. Only two combing evaluations are performed on each switch. The graders are asked to compare the treatments by combing with a narrow tooth nylon comb typical of those used by consumers and rate the ease/difficulty on a zero (ease) to ten (difficulty) scale. Ten separate evaluations are collected and the results analyzed by a statistical analysis package, such as Statgraphics Plus 5.1, for establishing statistical significance. Control charting is regularly used to insure that the low (Typical Clarifying Shampoo Formulation) and high (Typical Conditioning Shampoo Formulations) controls separate into regular domains. All conditioning prototypes must be more than two LSDs above the clarifying control to be viewed as acceptable.

For dry combing evaluations, the switches from above are moved into a controlled temperature and humidity room (22.2°C (72°F)/ 50%RH) and allowed to dry overnight. They remain separated as above and panelists are requested to evaluate dry conditioning performance by making three assessments; dry combing ease of the middle of the switch, dry combing ease of the tips, and a tactile assessment of tip feel. The same ten point scale [zero (ease) to ten (difficulty)] is used for these comparisons. Again, only two panelists make an assessment of each switch set. Statistical analysis to separate differences is done using the same method as above.

Hair Lather Rinsing Method

The hair lather rinse method is designed to assess the rinsing time for Typical Conditioning Shampoo Formulations under higher usage concentration, but at normal water temperature, water flow, and water quality. This allows greatest product differentiation. 4 gram, 8 inch switches are prewet for 10 seconds with water at 37.8°C (100°F), 7-10 gpg hardness at a flow rate of 0.65-0.70 gpm. The excess water is squeezed out to achieve a water content of 1g $H_2O$ to 1g of hair. 1.0 g of Typical Conditioning Shampoo Formulations is applied to the hair switch at the middle, the switch is folded in the middle and it is worked in a back and forth motion for 30 seconds to generate lather. For the concentrated shampoos of the present invention 0.5g of shampoo is applied. An evaluation of the lather amount and texture is recorded. The switch is unfolded and the water applied to the switch. A stop watch is started simultaneously and the time recorded for complete disappearance (water run clear) of lather on the switch. Each product is tested in triplicate and the average rinsing time determined. A typical test will analyze up to ten prototype products and an internal control, the clarifying shampoo example, is always included.

The rinsing times are compared to this control and superior rinsing for conditioning shampoos is comparable to this control and well below Typical Conditioning Shampoo Formulations.

The Typical Conditioning Shampoo Formulations in Example 4, column is used as the point of comparison. The Typical Conditioning Shampoo Formulations in Example 4 rinsing average time is indexed as 1.0. Rinsing indexes at 0.8 and below are judged to have an important rinsing advantage. The Typical Conditioning Shampoo Formulations indexes at about 0.65

METHOD:

Dissolution Method (shampoo time to dissolve)

*Experimental* - 0.5 mL of shampoo/product is placed on a typical glass slide (Corning 2947 MicroSlides). The shampoo/product covered slide is allowed equilibrate for 1 minute at ambient conditions. The glass slide containing product is then suspended in a beaker containing 1L of water (maintained @ 34°C +/-2°C). The solution is reproducibly stirred at 400 rpm (IKA BigSquid 1500 RPM Stirrer). The time is then recorded from when the slide is placed into the water until there is no visible shampoo/product on immersed slide. Results are given as seconds to dissolve.

METHOD: Rheological properties - shear

Equipment:

TA Instruments: ARES strain controlled rheometer equipped with fast-High speed DAQ module

Geometry: Cone & Plate

25 mm diameter - 0.04 rad cone

Gap = 0.051 mm
Temp= ambient

Test: Start-up of steady shear
Delay = 1.0 sec
Shear rate =1000 $s^{-1}$ for 5 sec
350 points collected

The rheometer is calibrated and as per operating instructions from manufacturer. The unit was fitted with a 25 mm diameter titanium cone and plate geometry. The temperature was ambient (ca. 22°C) with ambient humidity. The instrument was tarred for fixture mass and then zeroed for gap. The test is configured with the above conditions. A sample is loaded onto the lower plate (approx 0.5 g). The upper plate is lowered and the sample is seen to fill the gap completely. Once the gap of 0.051mm is achieved, the sample is trimmed with a plastic knife. The test is initiated via the computer. The method provides a 1 second delay (so the sample can rest after it is put into the apparatus) and then the shear rate of 1000 $s^{-1}$ ensues for 5 seconds. The instrument reaches the desired shear rate very rapidly (i.e., prior to the first data point collected).

The test method(s) discussed herein, including the Dissolution Method and the viscosity profile method, can be employed to evaluate consumer product compositions and component. A representative, non-limiting list of product categories includes antiperspirants, baby care, colognes, commercial products (including wholesale, industrial, and commercial market analogs to consumer-oriented consumer products), cosmetics, deodorants, dish care, feminine protection, hair care, hair color, health care, household cleaners, incontinence care, laundry, oral care, paper products, personal cleansing, disposable absorbent articles, pet health and nutrition, prescription drugs, prestige fragrances, skin care, snacks and beverages, special fabric care, shaving and other hair growth management products. Exemplary product forms and brands are described on The Procter & Gamble Company's website www.pg.com, and the linked sites found thereon. It is to be understood that one or more of said test methods may be useful for evaluating or measuring consumer products that are part of product categories other than those listed above are also contemplated.

Exemplary products within the laundry category include detergents, bleach, conditioners, softeners, anti-static products, and liquid refreshers. Exemplary products within the oral care category include dentifrice, denture adhesives, mouth rinses, gum care products, tooth whitening products, and other tooth care products. Exemplary baby care products include,wipes, and foaming bathroom hand soap. Exemplary health care products include oral and topical analgesics, gastro-intestinal treatment products, respiratory and cough/cold products, and water purification products. Exemplary hair care products include shampoos, conditioners (including rinse-off and leave-in forms), and styling aids. Exemplary household care products includes sweeper products, floor and surface cleaning products, wood floor cleaners, antibacterial floor and surface cleaners, fabric and air refreshers, and vehicle washing products. Skin care products include, but are not limited to, body washes, facial cleansers, hand lotions, moisturizers, conditioners, astringents, exfoliation products, microdermabrasion and peel products, skin rejuvenation products, anti-aging products, masks, UV protection

products, and wipes.

Pump Foam profile : foam longevity Method:

Fill a container having a foam-generating dispensers attached, such as WR-F3 series foamers from Airspray International, Inc., with the product. The product is dispensed from the container via the foam-generating dispenser.
The footprint area of the resulting foam in measured and the volume is approximated by measuring the height of the resulting foam. After waiting 2 minutes the measurements are repeated. The change in volume of the foam should be less than 50%, preferably less than 40%. If the change of volume of the foam is less than 50%, the pump foam is considered "good".

METHOD: ZPT Deposition Test Method

In Vitro Screening Test Method

This test method is designed for comparison of Zinc pyrithone (ZPT) deposition from different full product anti-dandruff formulations on the surface of pig skin, which is used as a surrogate for human scalp. The control treatment is a currently marketed formulation containing 1% ZPT. Two other formulations were added to the test to provide a range of deposition against which to judge any test formulations that may deposit at a higher coverage than the control. The 4% ZPT formulation contains a higher level of ZPT in the same chassis as the marketed control and has been consistently observed to deposit at a much higher level than the control. The formulations tested were developed to deliver the same ZPT coverage using only half the dose of a normal anti-dandruff shampoo. In a typical test, up to 9 separate formulations can be assessed for deposition against a control and a high depositing formula. The pig skin substrate is shaved free of hair, fat scraped to a uniform thickness, washed and irradiated to assure no microbial interference in the testing.

Treatment Procedure.

100 $cm^2$ pieces of the pig skin are stored at -20°C and thawed as needed. One square of pig skin is used for 4 extractions. The pigskin is washed with 0.3 mL of DAWN® dishwashing detergent, rinsed and air dried. A square of pigskin is placed on a stainless steel plate (fat side against the plate) and two hair switches are clipped over the top of the skin. Switches are carefully spread across the skin to cover the entire skin area with hair. A metered shower head apparatus delivers tap water at 4.92 L/min (±0.757 L/min) [1.3 gal/min (± 0.2 gal/min)] and 37.8°C (36.1°C-39.4°C) [100°F (± 3° F)], and the skin/hair is held approximately 10 inches below the shower head and wetted for 10 seconds. For regular formulations, a positive transfer pipette is used to deliver 1.6 mL of test product in a zig-zag pattern across hair and pigskin. For test formulations, 0.8 mL is delivered in the same manner. Hair and skin are lathered for 30 seconds then rinsed for 10 seconds. Hair switches are removed and pigskin is dried with a hair dryer for 60 seconds. Extraction and Analysis Procedure.
After removal of the hair four sites (3.14$cm^2$) on each of the two pig skin samples for each test treatment (eight sites in total) are extracted with an 80/20 mixture of 0.5M EDTA/ethanol. The extracts are analyzed for pyrithione content by HPLC. The average of the measurement for the eight sites is recorded. Data is analyzed using an independent, student's 2 tailed t-Test, at 90% confidence limits.
By selecting appropriate deposition aids, not only can the deposition of ZPT, but also of other benefit agents, be controlled across the across the benefit agent particle size ranging from about 5 nanometers to about 50 microns . In one embodiment, the deposition aid is a cationic polymer.

NON-LIMITING EXAMPLES

The shampoo compositions illustrated in the following Examples are prepared by conventional formulation and mixing methods. All exemplified amounts are listed as weight percents and exclude minor materials such as preservatives, color solutions, imagery ingredients, botanicals, and so forth, unless otherwise specified. All percentages are based on weight unless otherwise specified.

EXAMPLE 1

The formulas below do not contain Isotropic Enabler, and the surfactant is not present as an isotropic phase in the composition. As a result, they exhibit combinations of dissolution, stability, and lather performance issues. Examples A-C demonstrate formulations that can be made with current surfactant raw materials without the addition of solvents or hydrotropes. Note that example of 2X concentrated conditioning and clarifiying shampoos could not be prepared

directly by water evaporation as shown in the first two columns. The formulations in the first two columns have surfactants in the gel phase which will not dilute/dissolve in water.

Table 1

| Formulation/ Component | 2X Conditioning Shampoo | 2X Clarifying Shampoo | A | B | C |
|---|---|---|---|---|---|
| Anionics | | | | | |
| ALS | 12 | -- | -- | -- | -- |
| ALE3S | 20 | -- | -- | -- | -- |
| SLS | -- | 14 | 17.1 | 1.5 | 9.5 |
| SLE(3)S | -- | 14 | 9.4 | 23.5 | 16 |
| Co-surfactants | | | | | |
| CMEA | 3 | 1 | -- | -- | 1.25 |
| CAPB | -- | 4 | 2.3 | -- | -- |
| CocoB | -- | -- | -- | 3 | -- |
| Fatty alc. | 1.8 | -- | -- | -- | -- |
| Cationic Polymer | | | | | |
| Guar | -- | -- | 0.15 | 0.15 | 0.15 |
| PQ-10 | 1.0 | -- | -- | -- | -- |
| Opacifier | | | | | |
| EGDS | 1.5 | -- | 1.5 | 1.5 | 1.5 |
| Benefit Agents | | | | | |
| 35 $\mu$m dimethicone Silicone | 2.7 | -- | 2.1 | 2.1 | 2.1 |
| Perfume | 1 | -- | 1 | 1 | 1 |
| Finishing Agents[1] | qs | qs | qs | qs | qs |
| Performance Properties | | | | | |
| Dissolution | -- | -- | 220 seconds POOR | 125 seconds GOOD | 200 seconds ACCEPTA BLE |
| Lather Index | -- | -- | 0.97 OK = ACCEPTA BLE | 0.71 POOR | 0.31 POOR |
| Concentration Method | Not Possible | Not Possible | H2O evap. | H2O evp | H2O evp |
| [1] includes preservatives, pH control agents and water | | | | | |

Examples A-C show concentrated formulations made by evaporations prepared using traditional formulation approaches. Example A shows a 2X concentrated formulation made with a high SLS level. It has unacceptable dissolution, stability and rheology properties. Example B shows a formula with high SLE(3)S and cocobetaine co-surfactant. It has poor lather performance. Example C shows a formulation with high SLE(3)S and CMEA co-surfactant. It also has poor lather performance.

EXAMPLE 2

This example 2 demonstrates that it is possible to make formulas with acceptable, stability, and rheology by using additives such as solvent and hydrotrope, but with increased carbon content and other negatives such as lather: Changing the ratio of ethoxylated a non-ethoxylated surfactants along with the addition of solvents or hydrotropes can produce acceptable rheology properties but do not lead to formulations with acceptable lather properties.

Table 2

| Formulation/ Component | A | B | C |
|---|---|---|---|
| Anionics | | | |
| SLS | 15.5 | 1.4 | 13 |
| SLE(3)S | 14 | -- | 13 |
| SLE(1)S | -- | 26.3 | -- |
| Co-surfactants | | | |
| CMEA | 1 | -- | 0.9 |
| CAPB | 4 | 3.8 | 3.8 |
| Cationic Polymer | | | |
| PQ-10 | -- | 0.9 | 0.9 |
| Guar | 0.4 | -- | -- |
| Opacifier | | | |
| EGDS | 1.5 | 1.46 | 1.5 |
| Solvent / Hydrotrope | | | |
| SCS | 1.4 | 0.56 | 1.5 |
| Ethanol | -- | 7.2 | -- |
| Propylene glycol | 4.5 | -- | 4.1 |
| Benefit Agents | | | |
| 35 μm dimethicone Silicone | 2.7 | 2.6 | 2.1 |
| Perfume | 1.5 | 1.5 | 1.5 |
| Finishing Agents[1] | qs | qs | qs |
| Performance Properties | | | |
| Lather Index | 0.76 POOR | 0.52 POOR | 0.68 POOR |
| Conditioning | ++ | ++ | + |
| [1] includes preservatives, pH control agents and water<br>Key: CONDITIONING -- ++ = AT LEAST 2SD ABOVE; 2+'S OK, BUT ONE PLUS IS MARGINAL | | | |

EXAMPLE 3

Using commercially available isotropic surfactant pastes, without the described Isotropic Enablers, the formulas below have a combination of stability, and lather issues vs. current products: These examples represent formulations you can make with current surfactant raw materials without addition of solvents/hydrotropes. Variations in the ratio of ethoxylated and non-ethoxylated anionics do not produce formulations with acceptable lather and dissolution properties.

Table 3

| Formulation/ Component | A | B | C | D | E |
|---|---|---|---|---|---|
| Anionics | | | | | |
| SLS | 26.0 | 1.0 | 1.0 | 7.3 | 19.0 |
| SLE(3)S | -- | 21.9 | 23.8 | 18.0 | 6.7 |
| **Co-surfactants** | | | | | |
| CMEA | 2.0 | | 3.0 | 2.0 | 2.0 |
| CAPB | -- | 3.0 | -- | -- | -- |
| Cationic Polymer | | | | | |
| Guar | 0.2 | 0.2 | 0.2 | 0.2 | 0.2 |
| Opacifier | | | | | |
| EGDS | 1.0 | 1.0 | 1.0 | 1.0 | 1.0 |
| Benefit Agents | | | | | |
| LP-Silicone | 1.0 | 1.0 | 1.0 | 1.0 | 1.0 |
| Finishing Agents[1] | qs | qs | qs | qs | qs |
| Performance Properties | | | | | |
| Lather Index | 1.0 OK | 0.22 POOR | 0.17 POOR | 0.35 POOR | 0.83 OK |
| Wet Conditioning | ++ | ++ | ++ | ++ | ++ |
| Dry Conditioning | ++ | ++ | ++ | ++ | ++ |
| Dissolution | 225 seconds POOR | 125 seconds GOOD | 125 seconds GOOD | 200 seconds ACCEPTABL E | 220 seconds POOR |
| [1]includes preservatives, pH control agents and water | | | | | |

EXAMPLE 4

With our Isotropic Enablers, the concentrated formulas below have acceptable rheology, stability, and lather compared to a conditioning shampoo control similar to currently marked products. These formulations were prepared from isotropic pastes using typical mixing.

Table 4

| Formulation/ Component | Conditioning Shampoo Control | A | B | C | D | E | F | G |
|---|---|---|---|---|---|---|---|---|
| Anionics | | | | | | | | |
| ALS | 6 | -- | -- | -- | -- | -- | -- | -- |
| ALE(3)S | 10 | -- | -- | -- | -- | -- | -- | -- |
| SLS | -- | 1.5 | 1.5 | 1.5 | 1.25 | 1.25 | 1.25 | 3.65 |

(continued)

| Formulation/ Component | Conditioning Shampoo Control | A | B | C | D | E | F | G |
|---|---|---|---|---|---|---|---|---|
| SLE(1)S | -- | 16 | -- | 17 | 20 | 15 | 16.4 | 16.4 |
| C11S | -- | 8 | 8 | 5.2 | -- | -- | 5.0 | -- |
| C11E(1)S | -- | -- | 16 | -- | -- | -- | -- | -- |
| SLS w/ C10 | -- | -- | -- | -- | -- | 5 | -- | 5.0 |
| Cetearyl Sulfate | -- | -- | -- | 2.5 | -- | -- | -- | -- |
| Co-surfactants | | | | | | | | |
| CMEA | 1.5 | -- | -- | -- | -- | -- | -- | -- |
| CocoB | | 3 | 3 | 1.8 | 2.0 | 2.0 | 2.2 | 2.2 |
| Cetyl alcohol | 0.9 | -- | -- | -- | -- | -- | -- | -- |
| Cationic Polymer | | | | | | | | |
| Guar | -- | 0.15 | 0.15 | | 0.1 | 0.1 | 0.1 | 0.1 |
| PQ-10 | 0.5 | -- | -- | 0.4 | -- | -- | -- | -- |
| Opacifier | | | | | | | | |
| EGDS | 1.5 | 1.5 | 1.5 | 1.5 | 1.25 | 1.5 | 1.25 | 1.25 |
| Benefit Agents | | | | | | | | |
| LP Silicone | 1.35 | 2.1 | 2.1 | 2.0 | 1.5 | 1.5 | 1.5 | 1.5 |
| Perfume | 1.5 | 1.5 | 1.5 | 1.5 | 1.5 | 1.5 | 1.5 | 1.5 |
| Finishing Agents[1] | To 100 | To 100 | To 100 | To 100 | To 100 | To 100 | To 100 | To 100 |
| Performance Properties | | | | | | | | |
| Rheology (OK/BAD) | OK | OK | OK | OK | OK | OK | OK | OK |
| Lather Index | Control | 1.3 GOOD | 1.4 GOOD | 0.97 OK | 0.95 OK | 0.95 OK | 1.0 OK | 1.1 OK |
| Conditioning | ++ | ++ | +++ | ++ | ++ | ++ | ++ | ++ |
| Dissolution (sec) | 100 GOOD | 150 GOOD | NM | 170 GOOD | 170 GOOD | 175 GOOD | NM | NM |
| Rinsing | 1.0 | 0.67 | 0.63 | 0.70 | NM | NM | 0.67 | 0.75 |
| [1] includes preservatives, pH control agents and water<br>NM = NOT MEASURED | | | | | | | | |

Example 4A- More examples of Isotropic Enablers: With our Isotropic Enablers, the formulas below have acceptable rheology, stability, conditioning, and lather vs. current products. They are prepared by mixing conventional isotropic pastes. A similar performance profile can be observed at 10% active Isotropic Enabler with the exception of NEODOL 67S which has a poor lather and conditioning profile.

Table 5

| Formulation/ Component | H | I | J | K | L | M | N |
|---|---|---|---|---|---|---|---|
| SLS | 1.25 | 1.25 | 1.25 | 1.25 | 1.25 | 1.25 | 1.5 |
| SLE(1)S | 15 | 15 | 15 | 15 | 19 | 15 | 16 |
| C11S | -- | -- | -- | -- | -- | -- | 6 |
| Lial 23 | 5 | -- | -- | -- | -- | -- | -- |
| Neodol 23 | -- | -- | -- | -- | -- | 5 | -- |
| $C_{13-15}$Paraffin Sulfonate | -- | -- | 5 | -- | -- | -- | -- |
| $C_{14-17}$Paraffin Sulfonate | -- | -- | -- | 5 | -- | -- | -- |
| NSKKbr$C_{12}$ | -- | 5 | -- | -- | -- | -- | -- |
| Neodol 67 Sulfate | -- | -- | -- | -- | 2.4 | -- | -- |
| Cetearyl Sulfate | -- | -- | -- | -- | -- | -- | 1.0 |
| CocoB | 2.0 | 2.0 | 2.0 | 2.0 | 2.2 | 2.0 | 1.9 |
| Guar | 0.1 | 0.1 | 0.1 | 0.1 | 0.1 | 0.1 | -- |
| AM:TRI | -- | -- | -- | -- | -- | -- | 0.1 |
| LP-Silicone | 1.5 | 1.5 | 1.5 | 1.5 | 1.5 | 1.5 | 2.5 |
| Perfume | 1.5 | 1.5 | 1.5 | 1.5 | 1.5 | 1.5 | 1.5 |
| EGDS | 1.25 | 1.25 | 1.25 | 1.25 | 1.25 | 1.25 | 1.25 |
| Finishing Agents[1] | To 100 | To 100 | To 100 | To 100 | To 100 | To 100 | To 100 |
| Lather Index | 1.0 OK | 1.1 OK | 0.98 OK | 0.88 OK | 0.81 OK | 1.0 OK | 1.2 OK |
| Dissolution | PASS | PASS | PASS | PASS | PASS | PASS | PASS |
| Rheology | OK | OK | OK | OK | OK | OK | OK |
| Conditioning | NM | ++ | ++ | ++ | NM | NM | NM |
| Rinsing | NM | NM | NM | NM | NM | NM | 0.58 |

[1]includes preservatives, pH control agents and water

Note: Additional prototypes were prepared with the Isotropic Enablers (others than SLE(1)S and Neodol 67 Sulfate) at 10% concentration levels. Similar performance properties were observed as those recorded for the 5% concentration formulations shown above.

EXAMPLE 5

Anti-Dandruff Specific products containing Isotropic Enablers:

Table 6

| Formulation/ Component | ANTI-DANDRUFF SHAMPOO CONTROL | A | B | C |
|---|---|---|---|---|
| Anionics | | | | |
| SLS | 6 | 1.5 | 1.5 | 1.5 |
| SLE(3)S | 10 | -- | -- | -- |
| SLE(1)S | -- | 15 | 15 | 15 |
| C11S | -- | 5 | 5 | -- |
| SLS w/C10 | -- | -- | -- | 5 |

(continued)

| Formulation/ Component | ANTI-DANDRUFF SHAMPOO CONTROL | A | B | C |
|---|---|---|---|---|
| Co-surfactants | | | | |
| CMEA | 1.5 | -- | -- | -- |
| CocoB | -- | 2.1 | 2.1 | 2.1 |
| Cetyl alcohol | 0.9 | -- | -- | -- |
| Cationic Polymers | | | | |
| Guar | 0.5 | 0.1 | -- | 0.1 |
| AM:TRI | -- | -- | 0.1 | -- |
| Opacifier | | | | |
| EGDS | 1.5 | 1.5 | 1.5 | 1.5 |
| Benefit Agents | | | | |
| LP Silicone | 1.35 | -- | 1.0 | -- |
| DC1784 and/or DC1782 | -- | 1.0 | -- | 1.0 |
| ZPT | 1.0 | 2.0 | 1.0 | 2.0 |
| Zinc Carbonate | 1.6 | .15 | 0.15 | .15 |
| Finishing Agents[1] | Qs | qs | qs | qs |
| Performance Properties | | | | |
| Lather Index | .69 | 1.1 | 1.0 | 1.1 |
| Rinsing Index | 1.1 | 0.67 | Not measured | 0.69 |
| ZPT Deposition Index | 1.0 | 1.2 | 0.72 | 1.2 |

[1] includes preservatives, pH control agents and water
Note that the concentrated formulations provide similar anti-dandruff active deposition levels with enhancements in both later and rinsing performance.

EXAMPLE 6

These examples show that 3X concentrated, prototypes can be made from higher active pastes and enable isotropic surfactant mixtures at or above 36%. The examples below demonstrate lather, conditioning, and rinsing benefits are maintained.

Table 7

| Formulation/ Component | A | B | C | D | E | F | G |
|---|---|---|---|---|---|---|---|
| Anionics | | | | | | | |
| SLS | 1.0 | 1.0 | 1.0 | 1.0 | 1.0 | 1.0 | 1.0 |
| SLE(1)S | 9 | 9 | -- | -- | -- | 9 | 14.5 |
| C11S | 21 | -- | -- | 30 | -- | -- | -- |
| SLS w/ C10 | -- | 21 | 30 | -- | -- | -- | -- |
| PS 1:2:1 | -- | -- | -- | -- | 30 | 21 | -- |
| NSKK brC12 | -- | -- | -- | -- | -- | -- | 15.5 |
| Neo67S | 1.5 | 1.5 | 1.5 | 1.5 | 1.5 | 1.5 | 2 |
| Co-surfactants | | | | | | | |
| CocoB | 3 | 3 | 3 | 3 | 3 | 3 | 2 |

(continued)

| Formulation/ Component | A | B | C | D | E | F | G |
|---|---|---|---|---|---|---|---|
| Cationic Polymer | | | | | | | |
| AM:TRI | 0.15 | 0.15 | 0.15 | 0.15 | 0.15 | 0.15 | 0.15 |
| Opacifier | | | | | | | |
| EGDS | 1 | 1 | 1 | 1 | 1 | 1 | 1 |
| Benefit Agents | | | | | | | |
| DC 1872 | 1 | 1 | 1 | 1 | 1 | 1 | 1 |
| Perfume | 1.5 | 1.5 | 1.5 | 1.5 | 1.5 | 1.5 | 1.5 |
| Finishing Agents[1] | qs | qs | qs | qs | qs | qs | qs |
| Lather Index | 1.0 | 0.98 | 1.1 | 1.3 | 1.2 | 1.1 | 0.82 |
| Rinsing | 0.49 | 0.56 | 0.57 | 0.47 | 0.43 | 0.49 | 0.61 |
| Rheology | OK | OK | OK | OK | OK | OK | OK |
| Conditioning | ++ | ++ | ++ | ++ | ++ | ++ | ++ |
| [1] includes preservatives, pH control agents and water | | | | | | | |

Table 8

| | A | B | C |
|---|---|---|---|
| Ingredients | | | |
| Surfactants | | | |
| Neodol 1 Sulfate | 15% | 15% | 15% |
| SLE(1)S | 15% | 15% | 15% |
| Co-surfactants | | | |
| 1,2-decanediol | | | 3% |
| decylglyceryl ether | | 3% | |
| Beaulight SHAA (Sanyo) | 3% | | |
| Pearlizers/Stabilizers | | | |
| EGDS | 0.20% | 0.20% | 0.20% |
| Deposition polymers | | | |
| Guar | 0.20% | 0.20% | 0.20% |
| Benefit Agent | | | |
| DC-1784 silicone emulsion | 2% | 2% | 2% |
| Perfume | 2% | 2% | 2% |
| Finishing Agents[1] | qs. | qs. | qs. |
| Lather Index @ 1/3 dose | 0.99 | 0.91 | 1.03 |
| Conditioning @ 1/3 dose | | | |
| wet | +++ | +++ | ++ |
| dry | +++ | +++ | +++ |
| rheology | OK | OK | OK |

(continued)

| | A | B | C |
|---|---|---|---|
| rinsing | N/A | N/A | N/A |
| [1]includes preservatives, pH control agents and water | | | |

EXAMPLE 7

Bubble Size Control: Importantly, when formulas are compacted to high concentrations, the result is large, airy bubbles which may negatively affect consumer perception of lather. The researchers have identified additives for concentrated products which reduce bubble size while maintaining lather volume and rinsing advantages.

Table 9

| Formulation/ Component | CONDITIONING SHAMPOO Control | A | B | C | D |
|---|---|---|---|---|---|
| Anionics | | | | | |
| ALS | 6 | -- | -- | -- | -- |
| ALE(3)S | 10 | -- | -- | -- | -- |
| SLS | -- | 1.25 | 1.25 | 1.25 | 1.25 |
| SLE(1)S | -- | 16.4 | 16.4 | 16.4 | 16.4 |
| C11S | -- | 5 | 5 | 5 | 5 |
| Cetearyl Sulfate | -- | -- | 1 | 5.6 | -- |
| NEODOL 67 Sulfate | -- | -- | -- | -- | 1.0 |
| Co-surfactants | | | | | |
| CMEA | 1.5 | -- | -- | -- | -- |
| CocoB | -- | 2.2 | 2.2 | 2.2 | 2.2 |
| Cetyl alcohol | 0.9 | -- | -- | -- | -- |
| Cationic Polymer | | | | | |
| Guar | -- | 0.1 | 0.1 | 0.1 | 0.1 |
| PQ-10 | 0.5 | -- | -- | -- | -- |
| Opacifier | | | | | |
| EGDS | 1.5 | 1.25 | 1.25 | 1.25 | 1.25 |
| Benefit Agents | | | | | |
| LP Silicone | 1.35 | 1.5 | 1.5 | 1.5 | 1.5 |
| Perfume | 0.7 | 1.5 | 1.5 | 1.5 | 1.5 |
| Finishing Agents[1] | qs | qs | qs | qs | qs |
| Performance Properties | | | | | |
| Bubble Size | 0.007 $mm^2$ | 0.017 $mm^2$ | 0.012 $mm^2$ | 0.007 $mm^2$ | 0.010 $mm^2$ |
| Lather Index | 0.7 | 1.0 | 1.0 | NM | 0.96 |
| Rinsing | 1.0 | NM | 0.70 | NM | 0.78 |
| [1] includes preservatives, pH control agents and water | | | | | |

Examples B-D demonstrate that Cetearyl Sulfate and NEODOL® 67 Sulfate can both be used to reduce bubble size to levels comparable with currently marketed products producing creamier lather with improved consumer feel.

Example 8 - Carbon Footprint Reduction

Looking at the typical 1X Typical Conditioning Shampoo Formulations we can calculate the approximate weight percent organic carbon in the total composition as 13.1%. In this calculation the weight percent carbon in each of the main ingredients is determined and summed. The total weight percent of all ingredients in the formulation is 22%.

Prototype A in Example 4 (Table 4) represents the high end of the carbon utilization of our 2X prototypes. The total weight percent of actives in the formula is 32.4% and the organic carbon content is 18.7%. At the ½ level dosage the carbon utilization for this prototype is cut in half to 9.4%. One of our more efficient prototypes is shown in Example M of Example 4A (Table 5). Its total weight percent actives is 27.7%, and its organic carbon content is 15.5%. Therefore used at then level dosage its carbon utilization per task is reduced to 7.8%. This same analysis can be applied to the 3X prototype D Example 6 (Table 7). Its total weight percent actives is 39.2%. Its organic carbon content is 20.0%. Dosed at 1/3 the level results in an organic carbon utilization of 6.7%.

Example 9 - Pump Foamer Examples

In order to make conditioning shampoos that can be dispensed as a desirable foam one must be selective in the technology to be deployed as the prototype products must have very low viscosities relative to typical shampoos and our more compact examples above. The typical acceptable range is a liquid with a viscosity less than 100cps, more preferably between 30cps and 60cps.

Recognizing that several of of the Isotropic Enablers have low viscosities at high activities, it is possible to use them in conjunction with cationic polymers which facilitate deposition of small particle conditioning agents and small particle conditioning agents, e.g. <100 nanometers in size to formulate conditioning shampoos that will work from a pump foamer. The compositions in Table 9 are prepared by simply mixing the chosen ingredients and adjusting the composition to the desired pH.

Pump Foamer Examples

Table 10

| | A | B | C | D | E |
|---|---|---|---|---|---|
| Anionic Surfactant | | | | | |
| SLE(1)S | -- | 7 | -- | -- | -- |
| C11S | 30 | -- | 23 | -- | -- |
| Neo67S | 3.5 | 3.5 | 2.5 | 2.5 | 2.5 |
| SLS w/C10 | -- | 21.5 | -- | 23 | 23 |
| Co-surfactant | | | | | |
| cocoB | -- | -- | 1.5 | 1.5 | 0.75 |
| Conditioning Agents | | | | | |
| DC 1872 | 2 | 2 | 2 | 2 | 2 |
| Cationic Polymer | | | | | |
| AM:TRI | 0.15 | 0.15 | 0.15 | 0.15 | 0.15 |
| Guar | -- | -- | -- | -- | -- |
| Pump Foam | good | good | good | good | good |
| Lather index | NM | 0.71 | NM | 0.92 | 0.98 |
| Conditioning | NM | ++ | NM | ++ | NM |

Table 11

| | A | B | C |
|---|---|---|---|
| Ingredient | | | |
| Surfactant | | | |
| Neodol 1 Sulfate | 30% | | |
| $C_{13}/C_{14}/C_{15}$ Para. Sulf. | | 30% | |
| SLS* (20% $C_{10}$) | | | 30% |
| Conditioning agent | | | |
| DC-1782 Silicone microemulsion. | 2% | 2% | 2% |
| Deposition aid | | | |
| AM-TRI | | #### | #### |
| Guar | 0.15% | | |
| Perfume | 2% | #### | #### |
| Finishing Agents[1] | qs. | qs. | qs. |
| [1] includes preservatives, pH control agents and water | | | |

Table 12

| | A | B | C | D | E |
|---|---|---|---|---|---|
| Surfactants | | | | | |
| SLS | 1.5 | 1.5 | 5.7 | 1.5 | 8.5 |
| SLE(1)S | 16 | 16 | 16 | 16 | 16.5 |
| C11S | 6 | 6 | -- | 6 | 1.5 |
| C10S | -- | -- | 1.8 | -- | -- |
| Cocobetaine | 2 | 2 | 2 | 2 | -- |
| Cationic Polymers | | | | | |
| AM:TRI | 0.1 | 0.1 | 0.1 | 0.2 | 0.05 |
| Guar | 0.4 | 0.4 | 0.4 | 0.2 | 0.4 |
| Others | | | | | |
| EGDS | 1.5 | 1.5 | 1.5 | 1.5 | 1.5 |
| Dimethicone | 2.0 | 2.0 | 2.0 | 2 | 1.7 |
| Perfume | 1.5 | 0.75 | 1.5 | 1.5 | 1.5 |
| Finishing Agents[1]<br>Performance<br>Lather Index<br>Conditioning Index | qs<br><br>1.2<br>+ | qs<br><br>1.3<br>+ | qs<br><br>1.2<br>NM | qs<br><br>NM<br>++ | qs<br><br>NM<br>+ |
| [1]includes preservatives, pH control agents and water<br>NM = not measured | | | | | |

The following examples are "clarifying shampoo" formulations which generally provide the highest lather volume profiles. Evaluations are conducted according to the switch lather test method employing a typical clarifying shampoo as a high control. A typical conditioning shampoo formulation is used as a mid-range control that is typical of a conditioning

shampoo. The relative volumes produced are indexed to this control. The typical difference between these products is a 30% higher lather volume for the clarifying shampoo.

Typical Clarifying Shampoo Formulation

Table 13

| Raw Material | % |
|---|---|
| | (wt. / wt.) |
| SLE3S | 7.0000 |
| tetrasodium EDTA | 0.1400 |
| Citric Acid (Anhy.) | 1.1100 |
| Sodium Citrate (dihydrate) | 0.0000 |
| CMEA | 0.5000 |
| Kathon CG | 0.0300 |
| SLS | 7.0000 |
| DMDM Hydantoin | 0.1000 |
| cocoB | 2.0000 |
| NaCl | 0.7000 |
| Perfume | 0.4600 |
| Distilled Water | To 100.0000 |

Typical Conditioning Shampoo Formulation

Table 14

| Ingredient | Wt.% |
|---|---|
| Distilled Water | To 100 |
| PQ-10 | 0.5000 |
| ALE(3)S | 10.0000 |
| Polyox - Stock Soln.* | 2.0000 |
| Citric Acid (50% Soln.) | 0.0800 |
| Puresyn 6 | 0.3000 |
| Puresyn 3E20 | 0.1000 |
| Sodium Chloride | 0.5500 |
| Sodium Benzoate | 0.2500 |
| Disodium EDTA | 0.1270 |
| Sodium Citrate (dihydrate) | 0.4520 |
| Cetyl Alcohol | 0.9000 |
| CMEA | 0.9000 |
| EGDS | 1.5000 |
| Kathon CG | 0.0333 |

(continued)

| Ingredient | Wt.% |
|---|---|
| ALS | 6.0000 |
| Panthenol | 0.0536 |
| Panthenyl Ethyl Ether | 0.0300 |
| Perfume | 0.5500 |
| Polydimethylsiloxane Emulsion | 1.3500 |
| Total | 25.6759 |
| Polyox Premix | 24.3000 |
| Kathon CG | 0.0330 |
| Distilled Water | To 100 |
| *Polyox Premix Formulation | |

Several abbreviations are used throughout the application. Below is a key that sets forth what such abbreviations mean.

Key to Abbreviations

ALS- Ammonium Lauryl Sulfate
ALE(3)S- Ammounium Laureth(3) Sulfate
AM:TRI- Polyquat 76
SLS - Sodium Lauryl Sulfate
SLE(1)S- Sodium Laureth(1) Sulfate
SLE(3)S- Sodium laureth(3) Sulfate
SLS w/ $C_{10}$ - Sodium Lauryl Sulfate with 20% Decylsulfate
$C_{11}$S- Undecyl sulfate
$C_{11}$E(1)- Undeceth(1) sulfate
CAPB- Cocoamidopropyl Betaine
CocoB- Cocobetaine
CMEA- Cocomonoethanolamide
EGDS- Etheylene Glycol Distearate
Guar- Hydroxypropyltrimoinum guar (cationic)
LP-Silicone - Large Particle (>20 um) silicone
DC 1872 or Micro Silicone 1872 - Dow Corning Silicone Microemulsion
PQ-10 Polyquat-10
PS 1:2:1- $C_{13}$-$C_{15}$ Paraffin Sulfonate
NSKKbr$C_{12}$- Sodium salt of hydroxyethyl-2-dodecyl ether sulfate
SCS- Sodium Cumene Sulfonate
Neo67S or Neodol 67 Sulfate - Mixture of Linear and Branched Hexadecyl and Heptadecyl Alcohol Sulfates
Neodol 1 Sulfate - Mixture of Linear and Branched Hexadecyl and Heptadecyl with an average on 1 mole of ethoxylation per molecule
Neodol 23 Sulfate- Mixture of Linear and Branched Dodecyl and Tridecyl Alcohol Sulfates Lial 23- Mixture of Linear and Branched Dodecyl and Tridecyl Alcohol Sulfates
ZPT- Zinc pyridinethione
DADMAC - MERQUAT 100 or Polyquaternium 6
Puresyn 6 - Hydrogenated Polydecene (ex. ExxonMobile Chemical)
Puresyn 3E20 - Trimethylol Propane Tricaprylate / Tricaprate (ex. ExxonMobile Chemical)

## Claims

1. A concentrated personal cleansing composition comprising:

   a. greater than 23 wt% surfactant, wherein said surfactant is in an isotropic phase in said composition; and
   b. greater than 0.05 wt% conditioning agent

   wherein the composition that is substantially free of organic solvent and hydrotrope, has a lather index greater than 0.75, preferably from 0.75 to 1.5 and a dissolution value of less than 200 sec, preferably from 10 sec to 200 sec, more preferably from 100 sec to 200 sec;
   where said surfactant comprises an effective amount of Isotropic Enabler, selected from the group consisting of: branched and non-branched versions of decyl and undecyl alkyl sulfates which are either ethoxylated or non-ethoxylated; decyl alcohol modified lauryl sulfate; paraffin sulfonates with chain lengths ranging from $C_{13}$ to $C_{17}$; mixtures of linear and branched-chain alcohol sulfates with carbon chain lengths $C_{12}$ to $C_{17}$ which are ethoxylated or non-ethoxylated; sodium salts of branched, methyl-2-hydroxy-decyl ether sulfates, hydroxyethyl-2-dodecyl ether sulfates, hydroxyethyl-2-decyl ether sulfates; monoethoxylated lauryl alkyl sulfates; and mixtures thereof;
   wherein said surfactant comprises an additional surfactant selected from the group consisting of anionic surfactants;
   wherein said surfactant comprises a co-surfactant selected from the group consisting of amphoteric surfactants, zwitterionic surfactants, cationic surfactants, nonionic surfactants and mixtures thereof.

2. The concentrated personal cleansing composition according to claim 1, wherein the isotropic enabler is selected from the group consisting in straight-chain undecyl sulfates having the formula (I) $R_1$-$O(CH_2CHR_3O)_y$-$SO_3M$, branched-chain undecyl sulfates having the general formula (II) $CH_3$-$(CH_2)_z$-$CHR_2$-$CH_2$-$O(CH_2\ CHR_3O)_y$-$SO_3M$, or mixtures thereof, where $R_1$ of formula (I) represents $CH_3(CH_2)_{10}$, $R_2$ of formula (II) represents H or a hydrocarbon radical comprising 1 to 4 carbon atoms such that the sum of the carbon atoms in z of formula (II) and $R_2$ of formula (II) is 8, $R_3$ of formula (II) is H or $CH_3$, y of formulae (I) and (II) is 0 to 7, the average value of y of formulae (I) and (II) is about 1 when y of formulae (I) and (II) is not = 0, and M of formulae (I) and (II) is a mono-valent or di-valent, positively-charged cation.

3. The concentrated personal cleansing composition according to any preceding claims, wherein the isotropic enabler is selected from the group consisting in straight-chain and/or branched-chain undecyl alkoxy sulfates having the general formula (II) $CH_3$-$(CH_2)_z$ -$CHR_2$-$CH_2$-O-$(CH_2CHR_3O)_y$-$SO_3M$, where $R_2$ of formula (II) represents H or a hydrocarbon radical comprising 1 to 4 carbon atoms such that the sum of the carbon atoms in z of formula (II) and $R_2$ is 8, $R_3$ of formula (II) is H or $CH_3$, y of formula (II) is 1 to 7, y of formula (II) has an average value of 1 or less, and M of formula (II) is a monovalent, positively-charged cation such as ammonium, sodium, potassium and/or triethanolamine cation,etc. or divalent salts of magnesium with two surfactant anions.

4. The concentrated personal cleansing composition according to any preceding claims, wherein the additional surfactant is selected from the group consisting of alkyl and alkyl ether sulfates of the formula (III) $ROSO_3M$ and formula (IV) $RO(C_2H_4O)_xSO_3M$, wherein R of formulae (III) and (IV) is alkyl or alkenyl of from about 8 to about 18 carbon atoms, x of formula (IV) is 1 to 10, and M of formulae (III) and (IV) is a water-soluble cation such as ammonium, sodium, potassium, and triethanolamine cation or salts of the divalent magnesium ion with two anionic surfactant anions; preferably wherein the additional surfactant is selected from the group consisting of ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium trideceth sulfate, sodium tridecyl sulfate, sodium methyl lauroyl taurate, sodium methyl cocoyl taurate, sodium lauroyl isethionate, sodium cocoyl isethionate, sodium laurethsulfosuccinate, sodium laurylsulfosuccinate, sodium tridecyl benzene sulfonate, sodium dodecyl benzene sulfonate, and mixtures thereof.

5. The concentrated personal cleansing composition according to any preceding claims, wherein the amphoretic surfactant is selected from the group consisting in derivatives of aliphatic secondary and tertiary amines in which the aliphatic radical can be straight or branched chain and wherein one substituent of the aliphatic substituents contains from about 8 to about 18 carbon atoms and one contains an anionic water solubilizing group.

6. The concentrated personal cleansing composition according to any preceding claims, wherein the zwitterionic surfactant is selected from the group consisting in derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one substituent contains an anionic group; betaines, sulfobetaines, amidobetaines, amidosulfobetaines; and mixtures thereof.

7. The concentrated personal cleansing composition according to any preceding claims, wherein the cationic surfactant is selected from the group consisting of cationic surfactants derived from amines that are protonated at the pH of the formulation, cationic surfactants derived from fatty quaternary ammonium salts, and mixtures thereof.

8. The concentrated personal cleansing composition according to any preceding claims, wherein the nonionic surfactant is selected from the group consisting of lauryl dimethylamine oxide, cocodimethylamine oxide, cocoamidopropylamine oxide, laurylamidopropyl amine oxide, alkylpolyethoxylates, cocomonoethanol amide, cocodiethanol amide, lauroylmonoethanol amide, alkanoyl isopropanol amides, fatty alcohols, and 2-hydroxyalkyl methyl ethers, and mixtures thereof.

9. The concentrated personal cleansing composition according to claim 1, wherein the co-surfactant is selected from the group consisting of Cocomonoethanol Amide, Cocoamidopropyl Betaine, Laurylamidopropyl Betaine, Cocobetaine, lauryl betaine, lauryl amine oxide, sodium lauryl amphoacetate; alkyl glyceryl ethers, alkyl-di-glyceryl ethers, 1,2-alkyl cyclic sulfites, 1,2-alkyl cyclic carbonates, 1,2-alkyl-epoxides, alkyl glycidylethers, and alkyl-1,3-dioxolanes, wherein the alkyl group contains 6 to 14 carbon atoms in linear or branched configuration; 1,2- alkane diols where the total carbon content is from 6 to 14 carbon atoms linear or branched, methyl-2-hydroxy-decyl ethers, hydroxyethyl-2-dodecyl ether, hydroxyethyl-2-decyl ether, and mixtures thereof.

10. The composition of claim 1, wherein said conditioning agent is selected from the group consisting of silicones, oils, cationic polymers, and mixtures thereof.

11. An article of manufacture, wherein said article comprises:

a. a pump dispenser suitable for foaming;
b. a concentrated personal cleansing composition as defined in any preceding claims that is substantially free of organic solvent and hydrotrope, wherein said composition has a lather index greater than 0.75, a dissolution value of less than 200 sec, and comprises: greater than 30 wt% surfactant, wherein said surfactant is in the isotropic phase in said composition; and greater than 0.05 wt% conditioning agent.

**Patentansprüche**

1. Konzentrierte Körperreinigungszusammensetzung, umfassend:

a. mehr als 23 Gew.-% Tensid, wobei das Tensid sich in einer isotropen Phase in der Zusammensetzung befindet; und
b. mehr als 0,05 Gew.-% Konditioniermittel,

wobei die Zusammensetzung, die im Wesentlichen frei von organischem Lösungsmittel und Hydrotrop ist, einen Schaumindex von über 0,75, vorzugsweise von 0,75 bis 1,5 und einen Auflösungswert von weniger als 200 s, vorzugsweise von 10 s bis 200 s, mehr bevorzugt von 100 s bis 200 s aufweist;
wobei das Tensid eine wirksame Menge an isotropem Aktivierer umfasst, ausgewählt aus der Gruppe bestehend aus: verzweigten und nicht verzweigten Versionen von Decyl- und Undecylalkylsulfaten, die entweder ethoxyliert oder nicht ethoxyliert sind; decylalkohol-modifiziertes Laurylsulfat; Paraffinsulfonate mit Kettenlängen von $C_{13}$ bis $C_{17}$; Mischungen linearer und verzweigtkettiger Alkoholsulfate mit Kohlenstoffkettenlängen $C_{12}$ bis $C_{17}$, die ethoxyliert oder nicht ethoxyliert sind; Natriumsalze von verzweigten Methyl-2-hydroxy-decylethersulfaten, Hydroxyethyl-2-dodecylethersulfaten, Hydroxyethyl-2-decylethersulfaten; monoethoxylierten Laurylalkylsulfaten; und Mischungen davon;
wobei das Tensid ein weiteres Tensid umfasst, ausgewählt aus der Gruppe bestehend aus anionischen Tensiden;
wobei das Tensid ein Co-Tensid umfasst, ausgewählt aus der Gruppe bestehend aus amphoteren Tensiden, zwitterionischen Tensiden, kationischen Tensiden, nichtionischen Tensiden und Mischungen davon.

**2.** Konzentrierte Körperreinigungszusammensetzung nach Anspruch 1, wobei der isotrope Aktivierer ausgewählt ist aus der Gruppe bestehend aus geradkettigen Undecylsulfaten mit der Formel (I) $R_1$-O($CH_2CHR_3O)_y$-$SO_3M$, verzweigtkettigen Undecylsulfaten mit der allgemeinen Formel (II) $CH_3$-$(CH_2)_z$-$CHR_2$-$CH_2$-O($CH_2$ $CHR_3O)_y$-$SO_3M$, oder Mischungen davon, worin $R_1$ der Formel (I) für $CH_3(CH_2)_{10}$ steht, $R_2$ der Formel (II) für H oder ein Kohlenwasserstoffradikal steht, das 1 bis 4 Kohlenstoffatome derart umfasst, dass die Summe der Kohlenstoffatome in z von Formel (II) und $R_2$ von Formel (II) 8 ist, $R_3$ der Formel (II) H oder $CH_3$ ist, y von Formeln (I) und (II) 0 bis 7 ist, der Durchschnittswert von y von Formeln (I) und (II) etwa 1 ist, wenn y von Formeln (I) und (II) nicht = 0 ist, und M von Formeln (I) und (II) ein einwertiges oder zweiwertiges, positiv geladenes Kation ist.

**3.** Konzentrierte Körperreinigungszusammensetzung nach einem der vorstehenden Ansprüche, wobei der isotrope Aktivierer ausgewählt ist aus der Gruppe bestehend aus geradkettigen und/oder verzweigtkettigen Undecylalkoxysulfaten mit der allgemeinen Formel (II) $CH_3$-$(CH_2)_{z-}CHR_2$-$CH_2$-O-$(CH_2CHR_3O)_y$-$SO_3M$, worin $R_2$ von Formel (II) für H oder ein Kohlenwasserstoffradikal steht, das 1 bis 4 Kohlenstoffatome derart umfasst, dass die Summe der Kohlenstoffatome in z von Formel (II) und $R_2$ 8 ist, $R_3$ von Formel (II) H oder $CH_3$ ist, y von Formel (II) 1 bis 7 ist, y von Formel (II) einen Durchschnittswert von 1 oder weniger hat, und M von Formel (II) ein einwertiges, positiv geladenes Kation wie Ammonium, Natrium, Kalium und/oder Triethanolaminkation usw. oder zweiwertige Salze von Magnesium mit zwei Tensidanionen ist.

**4.** Konzentrierte Körperpflegezusammensetzung nach einem der vorstehenden Ansprüche, wobei das zusätzliche Tensid ausgewählt ist aus der Gruppe bestehend aus Alkyl- und Akylethersulfaten der Formel (III) $ROSO_3M$ und Formel (IV) $RO(C_2H_4O)_xSO_3M$, worin R von Formeln (III) und (IV) Alkyl oder Alkenyl von etwa 8 bis etwa 18 Kohlenstoffatomen ist, x von Formel (IV) 1 bis 10 ist, und M von Formeln (III) und (IV) ein wasserlösliches Kation, wie Ammonium, Natrium, Kalium, und Triethanolaminkation oder Salze des zweiwertigen Magnesiumions mit zwei anionischen Tensidanionen ist, wobei das zusätzliche Tensid vorzugsweise ausgewählt ist aus der Gruppe bestehend aus Ammoniumlaurylsulfat, Ammoniumlaurethsulfat, Triethylaminlaurylsulfat, Triethylaminlaurethsulfat, Triethanolaminlaurylsulfat, Triethanolaminlaurethsulfat, Monoethanolaminlaurylsulfat, Monoethanolaminlaurethsulfat, Diethanolaminlaurylsulfat, Diethanolaminlaurethsulfat, Laurinmonoglyceridnatriumsulfat, Natriumlaurylsulfat, Natriumlaurethsulfat, Kaliumlaurethsulfat, Natriumlaurylsarcosinat, Natriumlauroylsarcosinat, Laurylsarcosin, Cocoylsarcosin, Ammoniumcocoylsulfat, Ammoniumlauroylsulfat, Natriumcocoylsulfat, Natriumlauroylsulfat, Kaliumcocoylsulfat, Kaliumlaurylsulfat, Monoethanolamincocoylsulfat, Natriumtridecethsulfat, Natriumtridecylsulfat, Natriummethyllauroyltaurat, Natriummethylcocoyltaurat, Natriumlauroylisethionat, Natriumcocoylisethionat, Natriumlaurethsulfosuccinat, Natriumlaurylsulfosuccinat, Natriumtridecylbenzolsulfonat, Natriumdodecylbenzolsulfonat, und Mischungen davon.

**5.** Konzentrierte Körperpflegezusammensetzung nach einem der vorstehenden Ansprüche, wobei das amphotere Tensid ausgewählt ist aus der Gruppe bestehend aus Derivaten von aliphatischen sekundären und tertiären Aminen, bei denen das aliphatische Radikal gerad- oder verzweigtkettig sein kann, und wobei ein Substituent der aliphatischen Substituenten von etwa 8 bis etwa 18 Kohlenstoffatome enthält und einer eine anionische wasserlösende Gruppe enthält.

**6.** Konzentrierte Körperpflegezusammensetzung nach einem der vorstehenden Ansprüche, wobei das zwitterionische Tensid ausgewählt ist aus der Gruppe, bestehend aus Derivaten von aliphatischen quartären Ammonium-, Phosphonium- und Sulfoniumverbindungen, in denen die aliphatischen Radikale gerad- oder verzweigtkettig sein können, und wobei einer der aliphatischen Substituenten von etwa 8 bis etwa 18 Kohlenstoffatome enthält und ein Substituent eine anionische Gruppe enthält; Betainen, Sulfobetainen, Amidobetainen, Amidosulfobetainen; und Mischungen davon.

**7.** Konzentrierte Körperpflegezusammensetzung nach einem der vorstehenden Ansprüche, wobei das kationische Tensid ausgewählt ist aus der Gruppe bestehend aus kationischen Tensiden, die von Aminen abgeleitet sind, die bei dem pH-Wert der Formulierung protoniert sind, kationischen Tensiden, die von fetten quartären Ammoniumsalzen abgeleitet sind, und Mischungen davon.

**8.** Konzentrierte Körperpflegezusammensetzung nach einem der vorstehenden Ansprüche, wobei das nichtionische Tensid ausgewählt ist aus der Gruppe bestehend aus Lauryldimethylaminoxid, Cocodimethylaminoxid, Cocoamidopropylaminoxid, Laurylamidopropylaminoxid, Alkylpolyethoxylaten, Cocomonoethanolamid, Cocodiethanolamid, Lauroylmonoethanolamid, Alkanoylisopropanolamiden, Fettalkoholen und 2-Hydroxyalkylmethylethern, und Mischungen davon.

**9.** Konzentrierte Körperpflegezusammensetzung nach Anspruch 1, wobei das Co-Tensid ausgewählt ist aus der Gruppe bestehend aus Cocomonoethanolamid, Cocoamidopropylbetain, Laurylamidopropylbetain, Cocobetain, Laurylbetain, Laurylaminoxid, Natriumlaurylamphoacetat; Alkylglycerylethern, Alkyl-di-glycerylethern, cyclischen 1,2-Alkylsulfiten, cyclischen 1,2-Alkylcarbonaten, 1,2-Alkyl-epoxiden, Alkylglycidylethern und Alkyl-1,3-dioxolanen, wobei die Alkylgruppe 6 bis 14 Kohlenstoffatome in linearer oder verzweigter Konfiguration enthält; 1,2-Alkandiolen, wobei der Gesamtkohlenstoffgehalt von 6 bis 14 linearen oder verzweigten Kohlenstoffatomen beträgt, Methyl-2-hydroxy-decylethern, Hydroxyethyl-2-dodecylether, Hydroxyethyl-2-decylether, und Mischungen davon.

**10.** Zusammensetzung nach Anspruch 1, wobei das Konditioniermittel ausgewählt ist aus der Gruppe bestehend aus Silikonen, Ölen, kationischen Polymeren, und Mischungen davon.

**11.** Herstellungsartikel, wobei der Artikel Folgendes umfasst:

a. einen Pumpenspender, für Schaumerzeugung geeignet;
b. eine konzentrierte Körperpflegezusammensetzung, wie in einem der vorstehenden Ansprüche definiert, die im Wesentlichen frei von organischem Lösungsmittel und Hydrotrop ist, wobei die Zusammensetzung einen Schaumindex von über 0,75, einen Auflösungswert von weniger als 200 s aufweist und Folgendes umfasst: mehr als 30 Gew.-% Tensid, wobei das Tensid sich in der isotropen Phase in der Zusammensetzung befindet, und mehr als 0,05 Gew.-% Konditioniermittel.

## Revendications

**1.** Composition concentrée pour la toilette comprenant :

a. plus de 23 % en poids d'agent tensioactif, dans laquelle ledit agent tensioactif est dans une phase isotrope dans ladite composition ; et
b. plus de 0,05 % en poids d'agent de conditionnement

où la composition qui est essentiellement dépourvue de solvant organique et d'hydrotrope, a un indice de mousse supérieur à 0,75, de préférence de 0,75 à 1,5 et une valeur de dissolution inférieure à 200 s, de préférence de 10 s à 200 s, plus préférablement de 100 s à 200 s ;
où ledit agent tensioactif comprend une quantité efficace d'activateur isotrope, choisi de préférence dans le groupe constitué de : versions ramifiées et non ramifiées de sulfates de décyl et undécyl-alkyle qui sont ou éthoxylés ou non éthoxylés ; laurylsulfate à modification alcool décylique ; sulfonates de paraffine avec longueurs de chaîne allant de $C_{13}$ à $C_{17}$ ; mélanges de sulfates d'alcool linéaires et à chaîne ramifiée avec des longueurs de chaîne carbonée de $C_{12}$ à $C_{17}$ qui sont éthoxylés ou non éthoxylés ; sels de sodium de composés ramifiés, sulfates d'éther méthyl-2-hydroxy-décylique, sulfates d'éther hydroxyéthyl-2-dodécylique, sulfates d'éther hydroxyéthyl-2-décylique ; sulfates de lauryl-alkyle monoéthoxylés ; et des mélanges de ceux-ci ;
dans laquelle ledit agent tensioactif comprend un agent tensioactif supplémentaire choisi dans le groupe constitué d'agents tensioactifs anioniques ;
dans laquelle ledit agent tensioactif comprend un co-tensioactif choisi dans le groupe constitué d'agents tensioactifs amphotères, d'agents tensioactifs zwittérioniques, d'agents tensioactifs cationiques, d'agents tensioactifs non ioniques et leurs mélanges.

**2.** Composition concentrée pour la toilette selon la revendication 1, dans laquelle l'activateur isotrope est choisi dans le groupe constitué de sulfates d'undécyle à chaîne linéaire de formule (I) $R_1$-O($CH_2CHR_3O$)$_y$-$SO_3M$, de sulfates d'undécyle à chaîne ramifiée de formule générale (II) $CH_3$-($CH_2$)$_z$-$CHR_2$-$CH_2$-O($CH_2$ $CHR_3O$)$_y$-$SO_3M$, ou leurs mélanges, où $R_1$ de la formule (I) représente $CH_3(CH_2)_{10}$, $R_2$ de la formule (II) représente H ou un radical hydrocarbure comprenant 1 à 4 atomes de carbone de telle sorte que la somme des atomes de carbone dans z de la formule (II) et $R_2$ de la formule (II) vaut 8, $R_3$ de la formule (II) est H ou $CH_3$, y des formules (I) et (II) va de 0 à 7, la valeur moyenne de y des formules (I) et (II) vaut environ 1 lorsque y des formules (I) et (II) n'est pas = 0, et M des formules (I) et (II) est un cation chargé positivement monovalent ou divalent.

**3.** Composition concentrée pour la toilette selon l'une quelconque revendication précédente, dans laquelle l'activateur isotrope est choisi dans le groupe constitué d'alcoxysulfates d'undécyle à chaîne linéaire et/ou à chaîne ramifiée de formule générale (II) $CH_3$-($CH_2$)$_z$-$CHR_2$-$CH_2$-O-($CH_2CHR_3O$)$_y$-$SO_3M$, où $R_2$ de la formule (II) représente H ou un radical hydrocarbure comprenant 1 à 4 atomes de carbone de telle sorte que la somme des atomes de carbone

dans z de la formule (II) et $R_2$ vaut 8, $R_3$ de la formule (II) est H ou $CH_3$, y de la formule (II) va de 1 à 7, y de la formule (II) a une valeur moyenne de 1 ou moins, et M de la formule (II) est un cation chargé positivement monovalent tel qu'un cation ammonium, sodium, potassium et/ou triéthanolamine, etc. ou des sels divalents de magnésium avec deux anions d'agent tensioactif.

**4.** Composition concentrée pour la toilette selon l'une quelconque revendication précédente, dans laquelle l'agent tensioactif supplémentaire est choisi dans le groupe constitué de sulfates d'alkyle et d'alkyl-éther de formule (III) $ROSO_3M$ et de formule (IV) $RO(C_2H_4O)_xSO_3M$, dans laquelle R des formules (III) et (IV) est un alkyle ou alcényle allant d'environ 8 à environ 18 atomes de carbone, x de la formule (IV) va de 1 à 10, et M des formules (III) et (IV) est un cation hydrosoluble tel qu'un cation ammonium, sodium, potassium et triéthanolamine ou des sels de l'ion magnésium divalent avec deux anions d'agent tensioactif anionique ; de préférence dans laquelle l'agent tensioactif supplémentaire est choisi dans le groupe constitué de lauryl-sulfate d'ammonium, lauréthyl-sulfate d'ammonium, lauryl-sulfate de triéthylamine, lauréthyl-sulfate de triéthylamine, lauryl-sulfate de triéthanolamine, lauréthyl-sulfate de triéthanolamine, lauryl-sulfate de monoéthanolamine, lauréthyl-sulfate de monoéthanolamine, lauryl-sulfate de diéthanolamine, lauréthyl-sulfate de diéthanolamine, monoglycéride sulfate de sodium laurique, lauryl-sulfate de sodium, lauréthyl-sulfate de sodium, lauréthyl-sulfate de potassium, lauryl-sarcosinate de sodium, lauroyl-sarcosinate de sodium, lauryl-sarcosine, cocoyl-sarcosine, cocoyl-sulfate d'ammonium, lauroyl-sulfate d'ammonium, cocoyl-sulfate de sodium, lauroyl-sulfate de sodium, cocoyl-sulfate de potassium, lauryl-sulfate de potassium, cocoyl-sulfate de monoéthanolamine, tridécéthyl-sulfate de sodium, tridécyl-sulfate de sodium, méthyl-lauroyl-taurate de sodium, méthyl-cocoyl-taurate de sodium, lauroyl-iséthionate de sodium, cocoyl-iséthionate de sodium, lauréthyl-sulfosuccinate de sodium, lauryl-sulfosuccinate de sodium, tridécylbenzène-sulfonate de sodium, dodécylbenzène-sulfonate de sodium, et leurs mélanges.

**5.** Composition concentrée pour la toilette selon l'une quelconque revendication précédente, dans laquelle l'agent tensioactif amphotère est choisi dans le groupe constitué de dérivés d'amines secondaires et tertiaires aliphatiques dans lesquels le radical aliphatique peut être à chaîne linéaire ou ramifiée et dans laquelle un substituant des substituants aliphatiques contient d'environ 8 à environ 18 atomes de carbone et un substituant contient un groupe hydrosolubilisant anionique.

**6.** Composition concentrée pour la toilette selon l'une quelconque revendication précédente, dans laquelle l'agent tensioactif zwittérionique est choisi dans le groupe constitué de dérivés de composés quaternaires aliphatiques ammonium, phosphonium et sulfonium, dans lesquels les radicaux aliphatiques peuvent être à chaîne linéaire ou ramifiée, et dans laquelle un des substituants aliphatiques contient d'environ 8 à environ 18 atomes de carbone et un substituant contient un groupe anionique ; des bétaïnes, des sulfobétaïnes, des amidobétaïnes, des amidosulfobétaïnes ; et leurs mélanges.

**7.** Composition concentrée pour la toilette selon l'une quelconque revendication précédente, dans laquelle l'agent tensioactif cationique est choisi dans le groupe constitué d'agents tensioactifs cationiques dérivés d'amines qui sont protonées au pH de la formulation, d'agents tensioactifs cationiques dérivés de sels d'ammonium quaternaire gras, et leurs mélanges.

**8.** Composition concentrée pour la toilette selon l'une quelconque revendication précédente, dans laquelle l'agent tensioactif non ionique est choisi dans le groupe constitué d'oxyde de lauryl-diméthylamine, oxyde de cocodiméthylamine, oxyde de coco-amidopropylamine, oxyde de laurylamidopropyl-amine, alkylpolyéthoxylates, coco-monoéthanolamide, coco-diéthanolamide, lauroyl-monoéthanolamide, alcanoyl-isopropanol-amides, alcools gras, et éthers 2-hydroxyalkyl-méthyliques, et leurs mélanges.

**9.** Composition concentrée pour la toilette selon la revendication 1, dans laquelle le co-tensioactif est choisi dans le groupe constitué de coco-monoéthanolamide, coco-amidopropylbétaïne, laurylamidopropyl-bétaïne, cocobétaïne, lauryl-bétaïne, oxyde de laurylamine, lauryl-ampho-acétate de sodium ; éthers alkyl-glycéryliques, éthers alkyl-diglycéryliques, sulfites cyclique de 1,2-alkyle, carbonates cycliques de 1,2-alkyle, 1,2-alkyl-époxydes, éthers alkyl-glycidyliques et alkyl-1,3-dioxolanes, dans laquelle le groupe alkyle contient 6 à 14 atomes de carbone dans une configuration linéaire ou ramifiée ; 1,2-alcane-diols où la teneur totale en carbone va de 6 à 14 atomes de carbone linéaires ou ramifiés, éthers méthyl-2-hydroxy-décyliques, éther hydroxyéthyl-2-dodécylique, éther hydroxyéthyl-2-décylique, et leurs mélanges.

**10.** Composition selon la revendication 1, dans laquelle ledit agent de conditionnement est choisi dans le groupe constitué de silicones, huiles, polymères cationiques, et leurs mélanges.

**11.** Article manufacturé, où ledit article comprend :

a. un distributeur à pompe approprié pour faire mousser ;
b. une composition concentrée pour la toilette selon l'une quelconque des revendications précédentes qui est essentiellement dépourvue de solvant organique et d'hydrotrope, dans lequel ladite composition a un indice de mousse supérieur à 0,75, une valeur de dissolution inférieure à 200 s, et comprend : plus de 30 % en poids d'agent tensioactif, dans lequel ledit agent tensioactif est dans la phase isotrope dans ladite composition ; et plus de 0,05 % en poids d'agent de conditionnement.

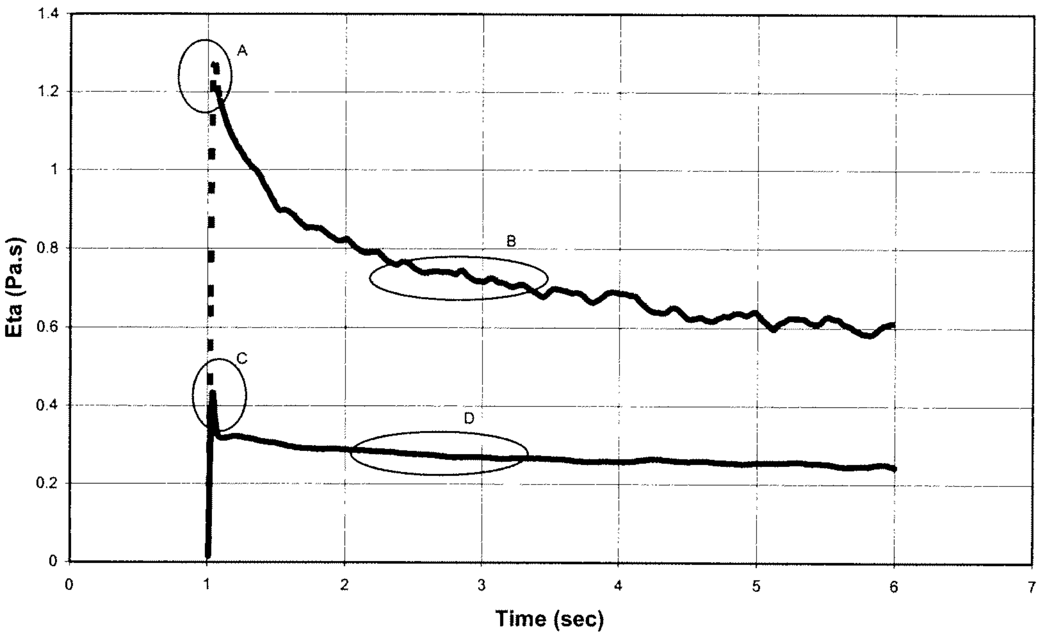


Figure 1

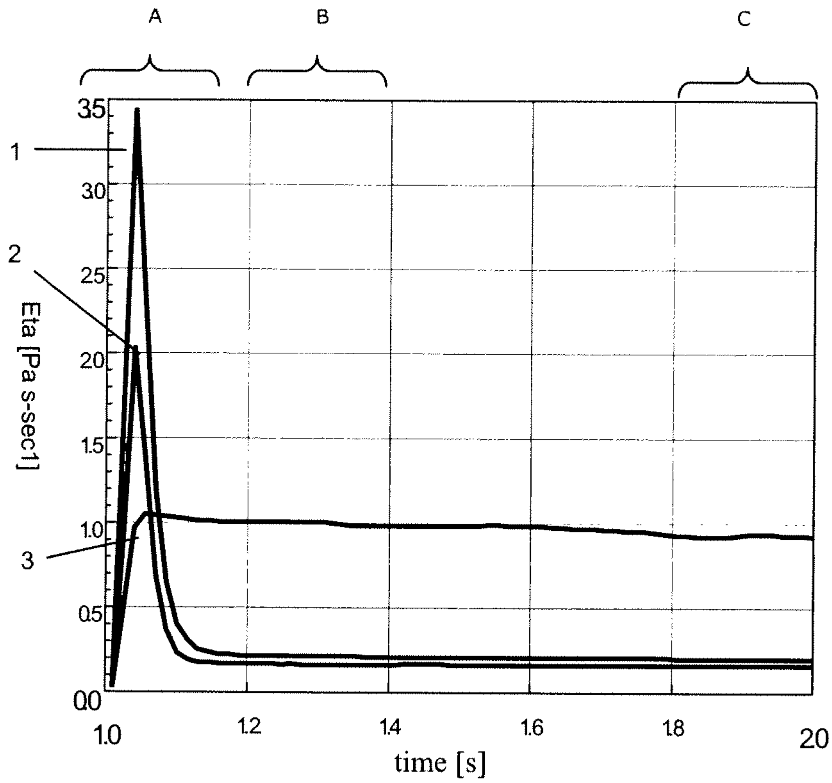


Figure 2

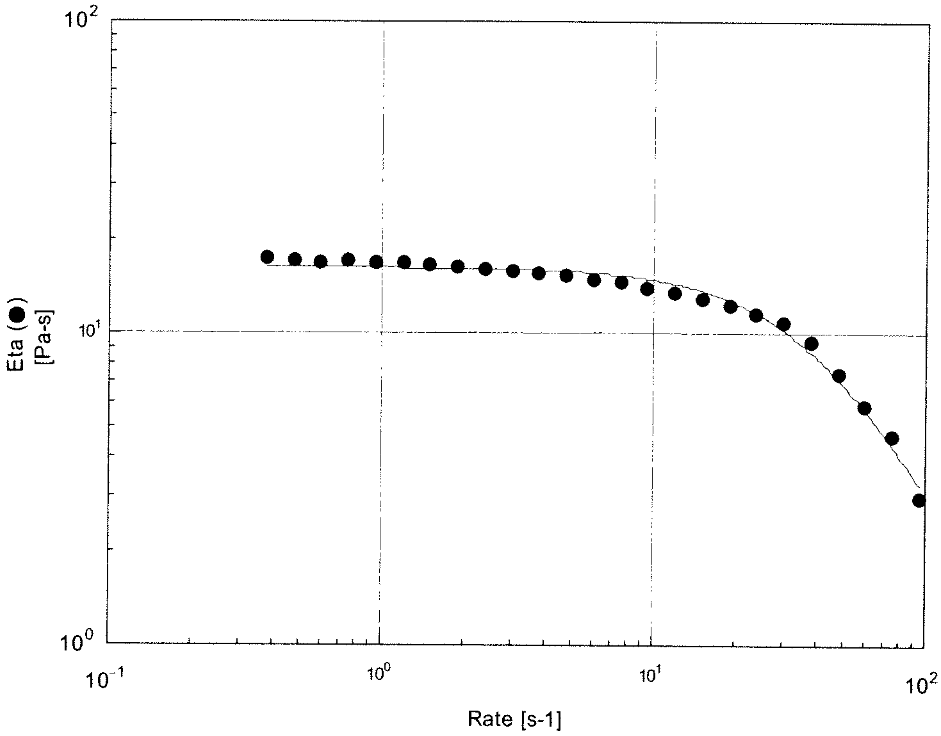


Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5942485 A
- EP 1696023 A1
- US 4753754 A
- JP 2002038200 A
- US 6706931 B
- US 5624666 A
- US 2658072 A
- US 2438091 A
- US 2528378 A
- US 5741948 A
- US 5994595 A
- US 6346509 B
- US 6417408 B
- US 3962418 A
- US 3958581 A
- US 20040010106 A1
- US 34584 A
- US 5104646 A
- US 5106609 A
- US 4152416 A
- US 5674478 A
- US 5750122 A
- US 4529586 A
- US 4507280 A
- US 4663158 A
- US 4197865 A
- US 4217914 A
- US 4381919 A
- US 4422853 A
- US 6335312 B
- US 2809971 A
- US 3236733 A
- US 3753196 A
- US 3761418 A
- US 4345080 A
- US 4323683 A
- US 4379753 A
- US 4470982 A
- US 2694668 A
- US 3152046 A
- US 4089945 A
- US 4885107 A
- WO 2004078903 A
- WO 2004078901 A
- WO 2005078063 A


### Non-patent literature cited in the description


- **MCCUTCHEON'S.** Detergents and Emulsifiers. Allured Publishing Corporation, 1986
- **MCCUTCHEON'S.** Functional Materials. 1992
- CTFA Cosmetic Ingredient Dictionary. The Cosmetic, Toiletry, and Fragrance Association, Inc, 1982
- **NOLL ; WALTER.** Chemistry and Technology of Silicones. Academic Press, 1968
- CTFA Cosmetic Ingredient Handbook. Cosmetic, Toiletry, and Fragrance Association, Inc, 2004
- **CREPALDI, EL ; PAVA, PC ; TRONTO, J ; VALIM, JB.** *J. Colloid Interfac. Sci.,* 2002, vol. 248, 429-42
- **MORIOKA, H. ; TAGAYA, H. ; KARASU, M ; KADOKAWA, J ; CHIBA, K.** *Inorg. Chem.,* 1999, vol. 38, 4211-6
- *Food & Package,* 2001, vol. 42 (10), 609-13
- *Food & Package,* 2001, vol. 42 (11), 676-79
- *Food & Package,* 2001, vol. 42 (12), 732-35